(19) Europäisches Patentamt
European Patent Office
Office européen des brevets

(11) **EP 4 159 251 A1**

(12) **EUROPEAN PATENT APPLICATION**
published in accordance with Art. 153(4) EPC

(43) Date of publication:
**05.04.2023 Bulletin 2023/14**

(21) Application number: **21817270.8**

(22) Date of filing: **01.06.2021**

(51) International Patent Classification (IPC):
**A61L 31/04** (2006.01)  **A61K 47/36** (2006.01)
**A61K 47/38** (2006.01)

(52) Cooperative Patent Classification (CPC):
**A61K 47/36; A61K 47/38; A61L 31/04**

(86) International application number:
**PCT/JP2021/020791**

(87) International publication number:
**WO 2021/246388 (09.12.2021 Gazette 2021/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.06.2020 JP 2020095280**

(71) Applicant: **Otsuka Pharmaceutical Factory, Inc.**
**Naruto-shi, Tokushima 772-8601 (JP)**

(72) Inventors:
• **OHHATA, Atsushi**
  **Naruto-shi, Tokushima 772-8601 (JP)**
• **YAMASHITA, Hiromichi**
  **Naruto-shi, Tokushima 772-8601 (JP)**
• **FUJIMOTO, Shiori**
  **Naruto-shi, Tokushima 772-8601 (JP)**

(74) Representative: **Hoffmann Eitle**
**Patent- und Rechtsanwälte PartmbB**
**Arabellastraße 30**
**81925 München (DE)**

(54) **ADHESION-PREVENTING AGENT AND METHOD FOR PREVENTING ADHESION USING SAME**

(57)    Provided are an adhesion preventing agent and an adhesion preventing method using the same. Specifically, provided are an adhesion preventing agent, including at least one anionized nanomaterial selected from the group consisting of anionized nanocellulose and anionized nanochitin, and an adhesion preventing method using the adhesion preventing agent.

Fig. 2

FIG. 2 (Example 18)

EP 4 159 251 A1

**Description**

BACKGROUND OF THE INVENTION

1. Field of the Invention

The present invention relates to an adhesion preventing agent and an adhesion preventing method using the same.

2. Description of the Related Art

Adhesion refers to a state in which tissue surfaces that should be separate from each other are connected or united via a fibrous tissue. Adhesions frequently occur after abdominal surgery, leading to adhesive intestinal obstruction, increased difficulty and risk of repeat surgery, reduced fertility, and chronic abdominal pain. In particular, a case of developing intestinal obstruction due to adhesion is life-threatening, and hence poses a clinically serious problem.

As means for preventing adhesion, a sheet-like adhesion preventing material (Seprafilm (trademark) or Interceed (trademark)) and a gel-like adhesion preventing material (AdSpray (trademark)) are commercially available. Each of those adhesion preventing materials is configured to reduce adhesion by being attached or sprayed to a site under an abdominal wall incision or to an injured part to provide a physical barrier between the incision or the injured part and an organ adjacent thereto.

There has been reported a systematic review that the sheet-like adhesion preventing material has been evidenced to reduce adhesion at its attachment site, but has not been evidenced to reduce the incidence of adhesive intestinal obstruction (for example, NPL 1). The gel-like adhesion preventing material is configured to exhibit an adhesion preventing effect by mixing two liquids and spraying the mixture around an injury site, immediately followed by gelling. However, preparation of the two liquids is complicated, and the adhesion preventing effect is limited to the application site. The reason why those existing adhesion preventing materials cannot completely prevent adhesive intestinal obstruction is because the materials are used only for a site under an abdominal wall incision or an injured part where adhesion is clearly predicted to occur.

In actuality, sites where adhesion occurs are indefinite, and exist over a wide range inside a peritoneal cavity. Accordingly, adhesion across the entire inside of the peritoneal cavity cannot be completely prevented through use of the existing adhesion preventing materials. Further, most cases of adhesive intestinal obstruction are caused by adhesion involving an bowel, especially a small intestine, where a site of adhesion occurrence is difficult to predict, and hence there is a demand for further improvements to adhesion preventing products.

In order to solve such clinical problems, adhesion needs to be prevented over a wide range inside the peritoneal cavity, including not only the site under an abdominal wall incision or the injured part where adhesion is clearly predicted to occur, but also the bowel, especially the small intestine, where a site of adhesion occurrence is difficult to predict.

In Example 19 of PTL 1, there is a description that an adhesion preventing effect was found by applying a pulp-derived nanocellulose aqueous dispersion liquid to a hepatic resection surface. In addition, in PTL 2, there is a description that tissue adhesion at an injury site can be minimized by applying a microbial cellulose material to the injury site.

In PTL 3 and NPL 2, there is a description that a hydrogel composition formed of a TEMPO-oxidized cellulose nanofiber (TOCN), methyl cellulose, carboxymethyl cellulose, and polyethylene glycol can be used to prevent peritoneal adhesion. There is a description that the gel composition has properties of being a transparent liquid at 4°C, but losing fluidity by gelling at 25°C and 37°C. Citation List

Patent Literature

PTL 1: WO 2015/099083 A1: Example 19
PTL 2: WO 2010/080264 A1
PTL 3: US 2019/0015564

Non Patent Literature

NPL 1: Intra-peritoneal prophylactic agents for preventing adhesions and adhesive intestinal obstruction after non-gynaecological abdominal surgery, Cochrane Database Syst Rev. 2009 Jan 21; (1): CD005080. doi: 10.1002/14651858.CD005080.pub2. NPL 2: Materials Science and Engineering C 102 (2019) 12-21

SUMMARY OF THE INVENTION

An object of the present invention is to provide a novel adhesion preventing agent and an adhesion preventing method using the same. Moreover, the object is to provide an adhesion preventing agent capable of preventing adhesion not only at an injury site or an inflammatory site in a tissue or an organ, where adhesion is predicted to occur, but over a wide range inside a peritoneal cavity, and an adhesion preventing method using the same.

The inventors have made extensive investigations in order to achieve the above-mentioned object, and as a result, have found that an aqueous dispersion liquid of anionized nanocellulose or anionized nanochitin can effectively prevent adhesion at an injury site or an inflammatory site. In addition, the inventors have also found that the aqueous dispersion liquid is free from thickening (maintains its fluidity without an increase in viscosity) near body temperature, and hence can be applied to a wide area including not only the injury site or the inflammatory site, but also the surroundings of the site inside a peritoneal cavity, thereby being able to prevent adhesion over a wide range inside the peritoneal cavity. Through further investigations based on such findings, the present invention has been completed.

That is, the present invention provides an adhesion preventing agent and an adhesion preventing method using the same.

Item 1. An adhesion preventing agent, including as an active ingredient at least one anionized nanomaterial selected from the group consisting of anionized nanocellulose and anionized nanochitin.

Item 2. The adhesion preventing agent according to Item 1, wherein the anionized nanomaterial is the anionized nanocellulose.

Item 3. The adhesion preventing agent according to Item 2, wherein the anionized nanocellulose is at least one member selected from the group consisting of an anionized cellulose nanofiber and an anionized cellulose nanocrystal.

Item 4. The adhesion preventing agent according to Item 2 or 3, wherein an anionic group contained in the anionized nanocellulose is at least one member selected from the group consisting of a carboxyl group, a carboxymethyl group, a phosphoric acid ester group and a sulfuric acid ester group.

Item 5. The adhesion preventing agent according to Item 1, wherein the anionized nanomaterial is the anionized nanochitin.

Item 6. The adhesion preventing agent according to Item 5, wherein the anionized nanochitin is at least one member selected from the group consisting of an anionized chitin nanofiber and an anionized chitin nanocrystal.

Item 7. The adhesion preventing agent according to Item 5 or 6, wherein an anionic group contained in the anionized nanochitin is at least one member selected from the group consisting of a carboxyl group, a carboxymethyl group, a phosphoric acid ester group and a sulfuric acid ester group.

Item 8. The adhesion preventing agent according to any one of Items 1 to 7, further including a liquid medium, wherein the anionized nanomaterial is dispersed in the liquid medium.

Item 9. The adhesion preventing agent according to Item 8, wherein the liquid medium is a medium containing water.

Item 10. The adhesion preventing agent according to Item 8 or 9, wherein a concentration of the anionized nanomaterial in the adhesion preventing agent is from 0.1 wt% to 10 wt%.

Item 11. The adhesion preventing agent according to any one of Items 8 to 10, wherein the adhesion preventing agent has a viscosity of from 0.5 mPa·s to 2,000 mPa·s at 37°C.

Item 12. The adhesion preventing agent according to any one of Items 8 to 11, wherein a content of the anionized nanomaterial in a total solid content in the adhesion preventing agent is 25 wt% or more.

Item 13. The adhesion preventing agent according to any one of Items 1 to 12, wherein the adhesion preventing agent is for use by application to one of an organ and a tissue inside a peritoneal cavity.

Item 14. At least one anionized nanomaterial selected from the group consisting of anionized nanocellulose and anionized nanochitin, for use in preventing adhesion of one of an organ and a tissue inside a peritoneal cavity.

Item 15. A use of at least one anionized nanomaterial selected from the group consisting of anionized nanocellulose and anionized nanochitin, for producing an adhesion preventing agent.

Item 16. At least one anionized nanomaterial selected from the group consisting of anionized nanocellulose and anionized nanochitin, for use as an adhesion preventing agent.

Item 17. An adhesion preventing method, including applying the adhesion preventing agent of any one of Items 1 to 13 to one of an organ and a tissue inside a peritoneal cavity.

Item 18. A method of producing an adhesion preventing agent, including mixing as an active ingredient at least one anionized nanomaterial selected from the group consisting of anionized nanocellulose and anionized nanochitin with a liquid medium.

The adhesion preventing agent according to at least one embodiment of the present invention can effectively prevent adhesion through application of the anionized nanomaterial serving as its active ingredient to a site where adhesion is to be prevented. The adhesion preventing agent according to at least one embodiment of the present invention may have the dosage form of a dispersion liquid (in particular, an aqueous dispersion liquid) containing the active ingredient. The dispersion liquid has appropriate fluidity with a small viscosity even near body temperature, and hence has an advantage of allowing the active ingredient to be brought into contact with or stuck to an entire tissue surface over a wide range inside the peritoneal cavity, including not only a site under an abdominal wall incision or an injured part where adhesion is clearly predicted to occur, but also the bowel (especially the small intestine) and the like, where the occurrence of adhesion is difficult to predict.

The adhesion preventing agent according to at least one embodiment of the present invention can, as described above, prevent adhesion over a wide range inside the peritoneal cavity not limited to the site under an abdominal wall incision or the injured part, and hence can prevent adhesion involving the bowel (especially the small intestine), which leads to adhesive intestinal obstruction, which poses a clinically serious problem.

The adhesion preventing agent according to at least one embodiment of the present invention can effectively prevent adhesion over a period of time required for the healing of an injury site, and hence can reduce a complication due to adhesion. The adhesion preventing agent is absorbed into the body after the healing, and hence does not induce new adhesion due to a foreign body reaction or the like.

The adhesion preventing agent according to at least one embodiment of the present invention typically has the dosage form of a dispersion liquid containing the active ingredient, and hence is easy to handle with no need to undergo complicated treatment (e.g., pre-mixing and/or preparation or form adjustment) before use. In addition, the dispersion liquid has appropriate fluidity from room temperature to near body temperature, and hence can be applied as it is (by dropping, spraying, coating, dipping, injection, or the like) to a wide range inside the peritoneal cavity including the surface of an injury site. Accordingly, the adhesion preventing agent is suitably used in minimally invasive endoscopic surgery as well as abdominal surgery.

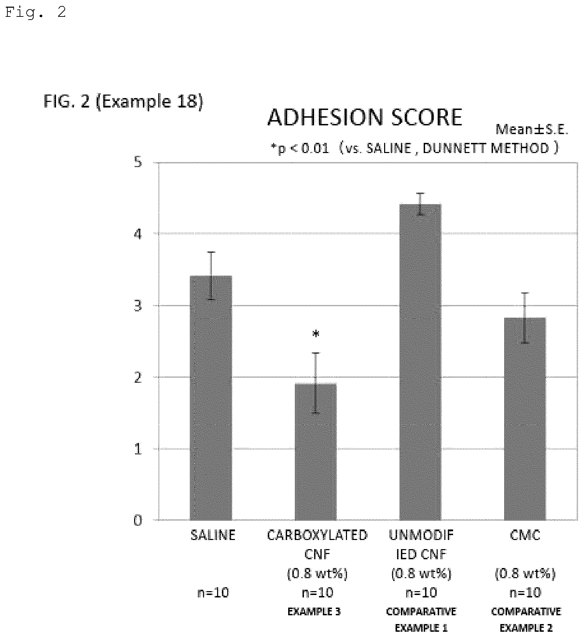

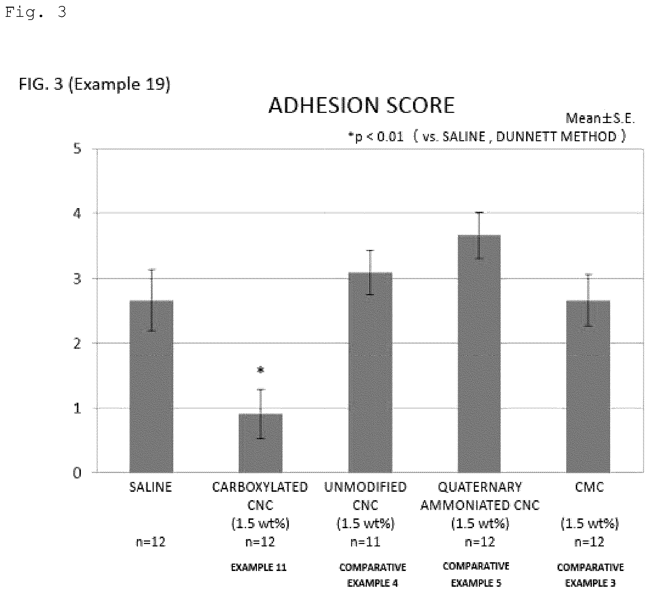

The adhesion preventing agent according to at least one embodiment of the present invention exhibits the above-mentioned excellent adhesion preventing effect presumably because of containing the anionized nanomaterial as the active ingredient. In contrast, an unmodified nanomaterial or a cationized nanomaterial cannot exhibit an adhesion preventing effect like that of at least one embodiment of the present invention, and may even aggravate adhesion (see FIG. 2 to FIG. 4 and FIG. 9, and Comparative Examples 1 and 4 to 7) . In addition, carboxymethyl cellulose (CMC) having cellulose molecular chains having an average diameter of subnanometer order (diameter: about 0.4 nm) cannot provide an excellent adhesion preventing effect like that of at least one embodiment of the present invention when having the dosage form of a solution even if anionized (see FIG. 2 and FIG. 3, and Comparative Examples 2 and 3).

BRIEF DESCRIPTION OF THE DRAWINGS

FIG. 1A, FIG. 1B, and FIG. 1C are micrographs of anionized nanomaterials, specifically a carboxylated CNF, a carboxylated CNC, and a carboxylated chitin NC, respectively.
FIG. 2 is a graph showing the results of an adhesion prevention test described in Example 18.
FIG. 3 is a graph showing the results of an adhesion prevention test described in Example 19.
FIG. 4 is a graph showing the results of an adhesion prevention test described in Example 20.
FIG. 5 includes graphs showing the results of an adhesion prevention test described in Example 21.
FIG. 6 is a graph showing the results of an adhesion prevention test described in Example 22.
FIG. 7 is a graph showing the results of an adhesion prevention test described in Example 23.
FIG. 8 is a graph showing the results of an adhesion prevention test described in Example 24.
FIG. 9 is a graph showing the results of an adhesion prevention test described in Example 25.
FIG. 10 is a graph showing the results of an adhesion prevention test described in Example 26.

DESCRIPTION OF THE EMBODIMENTS

Specific embodiments of the present invention are described in detail below.

1. Adhesion Preventing Agent

An adhesion preventing agent according to at least one embodiment of the present invention contains as an active ingredient at least one kind of anionized nanomaterial selected from the group consisting of: anionized nanocellulose; and anionized nanochitin. It is preferred that the adhesion preventing agent according to at least one embodiment of the present invention contain the anionized nanomaterial and a liquid medium, and have the dosage form of a dispersion liquid in which the anionized nanomaterial is dispersed in the liquid medium.

(1) Anionized Nanocellulose

The anionized nanocellulose among the anionized nanomaterials is described. In the cell wall of a plant, cellulose microfibrils (elementary fibrils) having an average fiber diameter of from about 3 nm to about 4 nm are present as a basic platform chemical (basic element). The cellulose microfibrils are each a single very fine fiber formed of a bundle of several tens of regularly assembled cellulose molecular chains (having an average diameter of about 0.4 nm), and the cellulose microfibrils assemble together to form the skeleton of the plant. In at least one embodiment of the present invention, the term "nanocellulose" refers to a product obtained from a material containing plant fibers (e.g., wood pulp) by unraveling the fibers to a nanosize level. The term "fiber diameter" as used herein means the diameter of a fiber.

The anionized nanocellulose according to at least one embodiment of the present invention is a product in which some of the hydroxy groups of glucose on cellulose molecular chains present on the surface of nanocellulose have been anionized, that is, chemically modified into anionic groups. Accordingly, the anionized nanocellulose is sometimes referred to as "anionically modified nanocellulose".

In general, nanocellulose may have different basic structures due to its production method, and may be classified on the basis of the difference into, for example, a fiber form, i.e., a cellulose nanofiber (hereinafter sometimes referred to as "CNF") and a crystal form, i.e., a cellulose nanocrystal (hereinafter sometimes referred to as "CNC").

The average fiber diameter of the anionized CNF according to at least one embodiment of the present invention is generally from 3 nm to 100 nm, preferably from 3 nm to 50 nm, more preferably from 3 nm to 10 nm. The average fiber length thereof is generally from 0.3 um to 200 $\mu$m, preferably from 0.5 um to 50 $\mu$m, more preferably from 0.5 $\mu$m to 10 $\mu$m. The aspect ratio thereof is generally 50 or more, preferably from 50 to 10,000, more preferably from 50 to 1,000. The term "average" as used herein means arithmetic average.

The average fiber diameter of the anionized CNC according to at least one embodiment of the present invention is generally from 3 nm to 100 nm, preferably from 3 nm to 50 nm, more preferably from 3 nm to 10 nm. The average fiber length thereof is generally from 100 nm to 400 nm, preferably from 100 nm to 300 nm, more preferably from 100 nm to 200 nm. The aspect ratio thereof is generally less than 50.

The anionized nanocelluloses according to at least one embodiment of the present invention are respective anionized products of the CNF and the CNC, and have the respective features of the CNF and the CNC. The anionized nanocellulose according to at least one embodiment of the present invention is a concept including both of an anionized cellulose nanofiber (hereinafter sometimes referred to as "anionized CNF") and an anionized cellulose nanocrystal (hereinafter sometimes referred to as "anionized CNC").

An anionic group contained in the anionized nanocellulose according to at least one embodiment of the present invention is a group capable of being deprotonated to be negatively charged, and examples thereof include a carboxyl group (-COOH), a sulfuric acid ester group ($-O-SO_3H$), a phosphoric acid ester group ($-O-PO_3H_2$), a carboxymethyl group ($-CH_2COOH$), a phosphorous acid ester group ($-P(OR)_3$ or $-P(O)(OR)_2$, wherein R is independently a hydrogen atom or an organic group such as an alkyl group.), and a nitric acid ester group ($-O-NO_2H$).

Preferred examples of the anionized nanocellulose according to at least one embodiment of the present invention may include nanocellulose having a carboxyl group (hereinafter sometimes referred to as "carboxylated nanocellulose"), nanocellulose having a sulfuric acid ester group (hereinafter sometimes referred to as "sulfated nanocellulose"), nanocellulose having a phosphoric acid ester group (hereinafter sometimes referred to as "phosphorylated nanocellulose"), and nanocellulose having a carboxymethyl group (hereinafter sometimes referred to as "carboxymethylated nanocellulose").

Any such nanocellulose may form a salt with a cationic atom or atomic group as required. Examples of the salt include a salt of an alkali metal (e.g., lithium, sodium, or potassium), an ammonium salt, and a salt with an organic ammonium (e.g., a tetraalkylammonium). Of those, a salt of an alkali metal is preferred, and a sodium salt is more preferred. The anionized nanocellulose according to at least one embodiment of the present invention is meant to encompass nanocellulose having the above-mentioned anionic group and a salt thereof.

The carboxylated nanocellulose has a structure in which the hydroxy group at the 6-position of glucose forming cellulose, or the hydroxy groups at the 2-position and the 3-position of the glucose are chemically modified into carboxyl groups. Examples thereof include: a product obtained by converting some of the hydroxy groups at the 6-position into carboxyl groups through oxidation (by, for example, an oxidation method using TEMPO); and a product obtained by converting some of the hydroxy groups at the 2-position and the 3-position into carboxyl groups through oxidation (by, for example, an oxidation method using sodium hypochlorite pentahydrate). The carboxylated nanocellulose may be produced in accordance or in conformity with a known method, such as a method described in Japanese Patent Application Laid-open No. 2008-001728, Japanese Patent Application Laid-open No. 2017-155024, WO 2018/230354 A1, or ACS Sustainable Chem Eng 2020, 8, 17800-17806.

The concentration of the carboxyl group (functional group introduction amount) in the carboxylated nanocellulose is generally from 0.1 mmol/g to 10 mmol/g, preferably from 0.1 mmol/g to 5 mmol/g, more preferably from 0.1 mmol/g to 2.5 mmol/g. The functional group introduction amount may be generally determined by subjecting an aqueous dis-

person liquid (slurry) containing the carboxylated nanocellulose to neutralization titration with an alkaline aqueous solution (e.g., an aqueous solution of sodium hydroxide). For example, the functional group introduction amount may be measured in accordance with the description of [Introduced Functional Group Content Measurement 1] in Examples.

The sulfated nanocellulose generally has a structure in which some of the hydroxy groups at the 2-position, the 3-position, and/or the 6-position of glucose forming cellulose present on the surface of nanocellulose are chemically modified into sulfuric acid. The sulfated nanocellulose may be produced in accordance or in conformity with a known method, such as a method described in WO 2018/131721 A1.

The concentration of the sulfuric acid ester group (functional group introduction amount) in the sulfated nanocellulose is generally from 0.1 mmol/g to 10 mmol/g, preferably from 0.1 mmol/g to 5 mmol/g, more preferably from 0.1 mmol/g to 2.5 mmol/g. The functional group introduction amount may be generally determined by subjecting an aqueous dispersion liquid (slurry) containing the sulfated nanocellulose to neutralization titration with an alkaline aqueous solution (e.g., an aqueous solution of sodium hydroxide). For example, the functional group introduction amount may be measured in accordance or in conformity with the description of [Introduced Functional Group Content Measurement 2] in Examples.

The phosphorylated nanocellulose generally has a structure in which some of the hydroxy groups at the 2-position, the 3-position, and/or the 6-position of glucose forming cellulose present on the surface of nanocellulose are chemically modified with phosphoric acid. The phosphorylated nanocellulose may be produced in accordance or in conformity with a known method, such as a method described in WO 2014/185505 A1.

The concentration of the phosphoric acid ester group (functional group introduction amount) in the phosphorylated nanocellulose is generally from 0.1 mmol/g to 10 mmol/g, preferably from 0.1 mmol/g to 5 mmol/g, more preferably from 0.1 mmol/g to 2.5 mmol/g. The functional group introduction amount may be measured, for example, in accordance or in conformity with the description of [Introduced Functional Group Content Measurement 2] in Examples.

The concentration of the carboxymethyl group (functional group introduction amount) in the carboxymethylated nanocellulose is generally from 0.06 mmol/g to 3.82 mmol/g, preferably from 0.3 mmol/g to 3.64 mmol/g, more preferably from 0.6 mmol/g to 3.44 mmol/g. The functional group introduction amount may be measured, for example, in accordance or in conformity with the description of [Introduced Functional Group Content Measurement 1] in Examples.

The adhesion preventing agent according to at least one embodiment of the present invention may contain one kind or a plurality of kinds of the anionized nanocelluloses. Of the anionized nanocelluloses, an anionized CNC is preferred over an anionized CNF from the viewpoint of further improving the adhesion preventing effect.

(2) Anionized Nanochitin

The anionized nanochitin among the anionized nanomaterials is described.

Chitin is a main component of: a cuticle covering the exoskeleton, that is, the outer shell of an arthropod or a crustacean, or the body surface of any of many invertebrates, such as the surface of the periostracum of a mollusk; the cell wall of a fungus, such as a mushroom; and the like. Chitin is a nitrogen-containing linear polysaccharide polymer, and means poly-$\beta$1-4-N-acetylglucosamine. Its structure is similar to the structure of cellulose, but has an acetamido group instead of a hydroxy group on the 2-position carbon. That is, chitin is a 1,4-polymer of N-acetylglucosamine. The term "nanochitin" refers to a product obtained by unraveling chitin obtained from the above-mentioned raw material to a nanosize level.

The anionized nanochitin according to at least one embodiment of the present invention is a product in which some of the hydroxy groups of N-acetylglucosamine present on the surface of nanochitin have been anionized, that is, chemically modified into anionic groups. Accordingly, the anionized nanochitin is sometimes referred to as "anionically modified nanochitin".

In general, nanochitin may have different basic structures due to its production method, and may be classified on the basis of the difference into, for example, a fiber form, i.e., a chitin nanofiber (hereinafter sometimes referred to as "chitin NF") and a crystal form, i.e., a chitin nanocrystal (hereinafter sometimes referred to as "chitin NC").

The average fiber diameter of the anionized chitin NF according to at least one embodiment of the present invention is generally from 3 nm to 100 nm, preferably from 3 nm to 50 nm, more preferably from 3 nm to 10 nm. The average fiber length thereof is generally from 0.5 um to 200 $\mu$m, preferably from 0.5 um to 50 $\mu$m, particularly preferably from 0.5 um to 10 um. The aspect ratio thereof is generally 50 or more, preferably from 50 to 10,000, more preferably from 50 to 1,000.

The average fiber diameter of the anionized chitin NC according to at least one embodiment of the present invention is generally from 3 nm to 100 nm, preferably from 3 nm to 50 nm, more preferably from 3 nm to 10 nm. The average fiber length thereof is generally from 100 nm to 400 nm, preferably from 100 nm to 300 nm, more preferably from 100 nm to 200 nm. The aspect ratio thereof is generally less than 50.

The anionized nanochitins according to at least one embodiment of the present invention are respective anionized products of the chitin NF and the chitin NC, and have the respective features of the chitin NF and the chitin NC. The anionized nanochitin according to at least one embodiment of the present invention is a concept including both of an anionized chitin nanofiber (hereinafter sometimes referred to as "anionized chitin NF") and an anionized chitin nanocrystal (hereinafter sometimes referred to as "anionized chitin NC").

An anionic group contained in the anionized nanochitin according to at least one embodiment of the present invention is a group capable of being deprotonated to be negatively charged, and examples thereof include a carboxyl group (-COOH), a sulfuric acid ester group ($-O-SO_3H$), a phosphoric acid ester group ($-O-PO_3H_2$), a carboxymethyl group ($-CH_2COOH$), a phosphorous acid ester group ($-P(OR)_3$ or $-P(O)(OR)_2$, wherein R is the same as above.), and a nitric acid ester group ($-O-NO_2H$). Of those, a carboxyl group is preferred.

Preferred examples of the anionized nanochitin according to at least one embodiment of the present invention may include nanochitin having a carboxyl group (hereinafter sometimes referred to as "carboxylated nanochitin"), nanochitin having a sulfuric acid ester group (hereinafter sometimes referred to as "sulfated nanochitin"), nanochitin having a phosphoric acid ester group (hereinafter sometimes referred to as "phosphorylated nanochitin"), and nanochitin having a carboxymethyl group (hereinafter sometimes referred to as "carboxymethylated nanochitin").

Any such nanochitin may form a salt with a cationic atom or atomic group as required. Examples of the salt include a salt of an alkali metal (e.g., lithium, sodium, or potassium), an ammonium salt, and a salt with an organic ammonium (e.g., a tetraalkylammonium). Of those, a salt of an alkali metal is preferred, and a sodium salt is more preferred. The anionized nanochitin according to at least one embodiment of the present invention is meant to encompass nanochitin having the above-mentioned anionic group and a salt thereof.

The carboxylated nanochitin generally has a structure in which some of the hydroxy groups at the 6-position of N-acetylglucosamine present on the surface of nanochitin are chemically modified into carboxyl groups. An example thereof is a product obtained by converting some of the hydroxy groups at the 6-position into carboxyl groups through oxidation (e.g., TEMPO oxidation). The carboxylated nanochitin may be produced in accordance or in conformity with a known method, such as a method described in Biomacromolecules 2008, 9, 192-198.

The concentration of the carboxyl group (functional group introduction amount) in the carboxylated nanochitin is generally from 0.1 mmol/g to 10 mmol/g, preferably from 0.1 mmol/g to 5 mmol/g, more preferably from 0.1 mmol/g to 2.5 mmol/g. The functional group introduction amount may be measured in accordance with, for example, the description of [Introduced Functional Group Content Measurement 1] in Examples.

The sulfated nanochitin generally has a structure in which some of the hydroxy groups at the 3-position and/or the 6-position of N-acetylglucosamine present on the surface of nanochitin are chemically modified with sulfuric acid. The sulfated nanochitin may be produced in accordance or in conformity with a known method, such as a method described in Carbohydrate Polymers, 2018, 197, 349-358.

The concentration of the sulfuric acid ester group (functional group introduction amount) in the sulfated nanochitin is generally from 0.1 mmol/g to 10 mmol/g, preferably from 0.1 mmol/g to 5 mmol/g, more preferably from 0.1 mmol/g to 2.5 mmol/g. The functional group introduction amount may be measured, for example, in conformity with the description of [Introduced Functional Group Content Measurement 2] in Examples.

The concentration of the phosphoric acid ester group (functional group introduction amount) in the phosphorylated nanochitin is generally from 0.1 mmol/g to 10 mmol/g, preferably from 0.1 mmol/g to 5 mmol/g, more preferably from 0.1 mmol/g to 2.5 mmol/g. The functional group introduction amount may be measured, for example, in conformity with the description of [Introduced Functional Group Content Measurement 2] in Examples.

The concentration of the carboxymethyl group (functional group introduction amount) in the carboxymethylated nanochitin is generally from 0.06 mmol/g to 3.82 mmol/g, preferably from 0.3 mmol/g to 3.64 mmol/g, more preferably from 0.6 mmol/g to 3.44 mmol/g. The functional group introduction amount may be measured, for example, in accordance or in conformity with the description of [Introduced Functional Group Content Measurement 1] in Examples.

The adhesion preventing agent according to at least one embodiment of the present invention may include one kind or a plurality of kinds of the anionized nanochitins. Of the anionized nanochitins according to at least one embodiment of the present invention, an anionized chitin NC is preferred, and a carboxylated chitin nanocrystal (hereinafter sometimes referred to as "carboxylated chitin NC") is more preferred.

(3) Liquid Medium

The liquid medium that may be contained in the adhesion preventing agent according to at least one embodiment of the present invention preferably contains pharmaceutically acceptable water. That is, the liquid medium is preferably an aqueous liquid medium.

Examples of the aqueous liquid medium include: waters, such as sterile water, purified water, distilled water, ion-exchanged water, and ultrapure water; aqueous solutions of electrolytes, such as saline, an aqueous solution of sodium chloride, an aqueous solution of potassium chloride, an aqueous solution of calcium chloride, an aqueous solution of magnesium chloride, an aqueous solution of sodium sulfate, an aqueous solution of potassium sulfate, an aqueous solution of sodium carbonate, an aqueous solution of sodium hydrogen carbonate, an aqueous solution of sodium acetate, and Ringer's solution; buffer solutions, such as a phosphate buffer solution and a Tris-hydrochloric acid buffer solution; aqueous solutions containing water-soluble organic substances, such as glycerin, ethylene glycol, ethanol, and isopropyl alcohol; aqueous solutions containing sugar molecules, such as glucose, sucrose, and maltose; aqueous solutions containing water-soluble polymers, such as polyethylene glycol and polyvinyl alcohol; aqueous solutions containing surfactants, such as octyl glucoside, dodecyl maltoside, and Pluronic (trademark) (polyethylene glycol/polypropylene glycol/polyethylene glycol copolymer) ; body fluids, such as intercellular fluid, extracellular fluid, interstitial fluid, lymph fluid, spinal fluid, blood, gastric juice, serum, plasma, saliva, tear, semen, and urine; and mixed liquids thereof. Of those, purified water, saline, Ringer's solution, and the like are preferred.

The adhesion preventing agent according to at least one embodiment of the present invention may further contain the following in addition to the above-mentioned components as required from the viewpoints of, for example, improving storage stability, promoting a therapeutic effect and an adhesion preventing effect, improving handleability, and preventing bacterial infection: pharmacological components, such as an antibacterial agent, an antibiotic, an anti-inflammatory agent, a blood circulation-improving agent, a steroid, an enzyme inhibitor, a growth factor, and various vitamins; additives, such as an excipient, a viscosity modifier, a gelling agent, a pH adjuster, a buffer, a preservative, an antioxidant, and a colorant; gas carriers, such as a nitrogen gas and a carbon dioxide gas; and the like.

(4) Dosage Form

The adhesion preventing agent according to at least one embodiment of the present invention is applied to the surface of a tissue or an organ inside a peritoneal cavity, and hence preferably has the dosage form of a dispersion liquid (in particular, an aqueous dispersion liquid) in which the anionized nanomaterial is dispersed in the liquid medium (in particular, aqueous liquid medium). The adhesion preventing agent according to at least one embodiment of the present invention may contain one kind or a plurality of kinds of the anionized nanomaterials. When the adhesion preventing agent according to at least one embodiment of the present invention is a dispersion liquid, the dispersion liquid contains the anionized nanomaterial in an amount effective for exhibiting an adhesion preventing effect. The concentration of the anionized nanomaterial in the adhesion preventing agent (dispersion liquid) is generally from 0.01 wt% to 10 wt%, preferably from 0.1 wt% to 5 wt%, more preferably from 0.2 wt% to 3 wt%, particularly preferably from 0.3 wt% to 2 wt% with respect to the total amount of the adhesion preventing agent (dispersion liquid).

The adhesion preventing agent according to at least one embodiment of the present invention contains the anionized nanomaterial as an active ingredient for preventing adhesion, preferably consists essentially of the anionized nanomaterial in terms of active ingredient, and more preferably consists only of the anionized nanomaterial in terms of active ingredient. The content of the anionized nanomaterial in the total solid content in the adhesion preventing agent is generally 25 wt% or more, preferably from 30 wt% to 100 wt%, more preferably from 30 wt% to 80 wt%, particularly preferably from 30 wt% to 60 wt%. When the content of the anionized nanomaterial in the total solid content is less than 100 wt%, the adhesion preventing agent according to at least one embodiment of the present invention contains an additive, such as an electrolyte or a sugar.

When the adhesion preventing agent according to at least one embodiment of the present invention is a dispersion liquid, the dispersion liquid preferably has appropriate fluidity in the range of from ordinary temperature to near body temperature from the viewpoints of operability and applicability to a wide range inside a peritoneal cavity. For example, the viscosity of the adhesion preventing agent (dispersion liquid) is generally from 0.5 mPa·s to 2,000 mPa·s, preferably from 0.5 mPa·s to 1,000 mPa·s, when its temperature is from 0°C to 40°C (further, from 25°C to 37°C, in particular, near 37°C). In particular, in the case of an anionized nanocrystal, the viscosity is more preferably from 0.5 mPa·s to 100 mPa·s. With such viscosity, the adhesion preventing agent (dispersion liquid) can be brought into contact with or stuck to an entire tissue surface inside the peritoneal cavity, including not only an injury site where adhesion is predicted to occur, but also a site where the occurrence of adhesion is difficult to predict. The viscosity may be measured using a rotary viscometer as described in Examples.

The pH of the adhesion preventing agent according to at least one embodiment of the present invention is generally from 4 to 8, preferably from 6 to 7.5, from the viewpoint of safety for a living body.

The adhesion preventing agent according to at least one embodiment of the present invention may have, for example, the following dosage form obtained from the above-mentioned dispersion liquid: a solution; a spray, such as an aerosol or a pump spray; a coating; or an injection. Depending on its form of use, the adhesion preventing agent may be directly applied onto a tissue, and may be formulated into a dosage form having good operability.

The adhesion preventing agent according to at least one embodiment of the present invention may be a composition further containing a pharmaceutically acceptable carrier. The pharmaceutically acceptable carrier may be selected in accordance with the dosage form to be used, and may be, for example, a gas carrier or a liquid carrier.

(5) Preferred Modes

As an adhesion preventing agent according to at least one preferred embodiment of the present invention, there is given an adhesion preventing agent that is an aqueous dispersion liquid containing an anionized nanomaterial and a liquid medium (in particular, an aqueous liquid medium), wherein the anionized nanomaterial is carboxylated nanocellulose (in particular, a carboxylated CNC) having a carboxyl group concentration of from 0.1 mmol/g to 2.5 mmol/g, wherein the concentration of the carboxylated nanocellulose in the adhesion preventing agent is from 0.3 wt% to 2 wt%, wherein the content of the anionized nanomaterial in the total solid content in the adhesion preventing agent is from 30 wt% to 100 wt%, and wherein the adhesion preventing agent has a viscosity of from 0.5 mPa·s to 2,000 mPa·s (in particular, from 0.5 mPa·s to 1,000 mPa·s) at a temperature of from 25°C to 37°C (in particular, near 37°C) .

As an adhesion preventing agent according to at least one other preferred embodiment of the present invention, there is given an adhesion preventing agent that is an aqueous dispersion liquid containing an anionized nanomaterial and a liquid medium (in particular, an aqueous liquid medium), wherein the anionized nanomaterial is sulfated nanocellulose (in particular, a sulfated CNC) having a sulfuric acid ester group concentration of from 0.1 mmol/g to 2.5 mmol/g, wherein the concentration of the sulfated nanocellulose in the adhesion preventing agent is from 0.3 wt% to 2 wt%, wherein the content of the anionized nanomaterial in the total solid content in the adhesion preventing agent is from 30 wt% to 100 wt%, and wherein the adhesion preventing agent has a viscosity of from 0.5 mPa·s to 2,000 mPa·s (in particular, from 0.5 mPa·s to 1,000 mPa·s) at a temperature of from 25°C to 37°C (in particular, near 37°C) .

As an adhesion preventing agent according to at least one other preferred embodiment of the present invention, there is given an adhesion preventing agent that is an aqueous dispersion liquid containing an anionized nanomaterial and a liquid medium (in particular, an aqueous liquid medium), wherein the anionized nanomaterial is phosphorylated nanocellulose (in particular, a phosphorylated CNC) having a phosphoric acid ester group concentration of from 0.1 mmol/g to 2.5 mmol/g, wherein the concentration of the phosphorylated nanocellulose in the adhesion preventing agent is from 0.3 wt% to 2 wt%, wherein the content of the anionized nanomaterial in the total solid content in the adhesion preventing agent is from 30 wt% to 100 wt%, and wherein the adhesion preventing agent has a viscosity of from 0.5 mPa·s to 2,000 mPa·s (in particular, from 0.5 mPa·s to 1,000 mPa·s) at a temperature of from 25°C to 37°C (in particular, near 37°C) .

As an adhesion preventing agent according to at least one other preferred embodiment of the present invention, there is given an adhesion preventing agent that is an aqueous dispersion liquid containing an anionized nanomaterial and a liquid medium (in particular, an aqueous liquid medium), wherein the anionized nanomaterial is carboxymethylated nanocellulose (in particular, a carboxymethylated CNC) having a carboxymethyl group concentration of from 0.06 mmol/g to 3.82 mmol/g, wherein the concentration of the carboxymethylated nanocellulose in the adhesion preventing agent is from 0.3 wt% to 2 wt%, wherein the content of the anionized nanomaterial in the total solid content in the adhesion preventing agent is from 30 wt% to 100 wt%, and wherein the adhesion preventing agent has a viscosity of from 0.5 mPa·s to 2,000 mPa·s (in particular, from 0.5 mPa·s to 1,000 mPa·s) at a temperature of from 25°C to 37°C (in particular, near 37°C) .

As an adhesion preventing agent according to at least one other preferred embodiment of the present invention, there is given an adhesion preventing agent that is an aqueous dispersion liquid containing an anionized nanomaterial and a liquid medium (in particular, an aqueous liquid medium), wherein the anionized nanomaterial is carboxylated nanochitin (in particular, a carboxylated chitin NC) having a carboxyl group concentration of from 0.1 mmol/g to 2.5 mmol/g, wherein the concentration of the carboxylated nanochitin in the adhesion preventing agent is from 0.3 wt% to 2 wt%, wherein the content of the anionized nanomaterial in the total solid content in the adhesion preventing agent is from 30 wt% to 100 wt%, and wherein the adhesion preventing agent has a viscosity of from 0.5 mPa·s to 2,000 mPa·s (in particular, from 0.5 mPa·s to 1,000 mPa·s) at a temperature of from 25°C to 37°C (in particular, near 37°C) .

2. Preparation of Adhesion Preventing Agent

The adhesion preventing agent according to at least one embodiment of the present invention preferably contains the anionized nanomaterial and the liquid medium, and other components may be further added as required as long as the adhesion preventing effect of at least one embodiment of the present invention can be exhibited. The adhesion preventing agent may be generally prepared by mixing the anionized nanomaterial with the liquid medium. Specifically, the adhesion preventing agent may be prepared by uniformly dispersing the anionized nanomaterial in the liquid medium. The adhesion preventing agent may be prepared by performing the uniform dispersion through use of, for example, an ultrasonic dispersion method or a stirring method (e.g., a homomixer) as a dispersion method.

The adhesion preventing agent according to at least one embodiment of the present invention is applied to, for example, an organ or a tissue inside a peritoneal cavity, and hence is preferably subjected to sterilization treatment. For the sterilization treatment, there may be adopted a known method, such as high-pressure steam sterilization, filter sterilization, or electron beam sterilization.

3. Method of Using Adhesion Preventing Agent

The adhesion preventing agent according to at least one embodiment of the present invention can effectively prevent adhesion by being used (applied) at a site where adhesion is to be prevented.

An example of the site where adhesion is to be prevented is an inflammatory site or an injury site in an organ, a tissue, or the like of a human or an animal. Particular examples thereof include a surgical incision site, a site of injury artificially caused by a procedure during surgery, and an endogenous or exogenous inflammatory site in a body. Examples of the organ include a stomach, a small intestine, a large intestine, a duodenum, a mesentery, a peritoneum, a cecum, a liver, a heart, a pericardium, a lung, a brain, an ovary, a uterus, a spleen, a greater omentum, and an eyeball. Examples of the tissue include a bone, a vertebra, a nerve, cartilage, a ligament, a tendon, a skin, a tube (e.g., a blood vessel or an oviduct), and fat.

When the adhesion preventing agent according to at least one embodiment of the present invention has the dosage form of a dispersion liquid (for example, an aqueous dispersion liquid) having appropriate fluidity with a small viscosity, adhesion can be prevented by allowing the active ingredient to be brought into contact with or stuck to an entire tissue surface over a wide range inside a peritoneal cavity, including not only the above-mentioned site under an abdominal wall incision or injured part where adhesion is clearly predicted to occur, but also the bowel, especially the small intestine, where a site of adhesion occurrence is difficult to predict.

The adhesion preventing agent according to at least one embodiment of the present invention may have the dosage form of a dispersion liquid (e.g., an aqueous dispersion liquid), and may be generally formulated into a solution, a spray (e.g., an aerosol or a pump spray), a coating, an injection, or the like. The formulation may be administered to the surface of the site where adhesion is to be prevented by dropping, exposure, spraying, coating, or the like.

The use amount of the adhesion preventing agent is not particularly limited as long as the amount allows its adhesion preventing effect to be exhibited. The adhesion preventing agent (dispersion liquid) may be applied in such a manner that the site where adhesion is to be prevented is coated with the adhesion preventing agent.

The adhesion preventing agent according to at least one embodiment of the present invention can be applied as it is to the site where adhesion is to be prevented, without undergoing complicated pretreatment, such as pre-use preparation required for an adhesion preventing agent of a two-liquid dosage form or shape or size adjustment required for a sheet-like adhesion preventing material, and hence is excellent in handleability. Accordingly, the adhesion preventing agent is suitably used particularly in minimally invasive endoscopic surgery.

The term "comprise", "include", "contain", or "have" as used herein is to be construed as encompassing "consist essentially of" and "consist only of" as well.

Examples

The present invention is specifically described below by way of Examples, but the present invention is not limited thereto.

Materials used in Examples and Comparative Examples are described. The meanings of abbreviations used in the following Examples and Comparative Examples are as shown below.

CNF: cellulose nanofiber
CNC: cellulose nanocrystal
CMC: carboxymethyl cellulose
Chitin NC: chitin nanocrystal
Chitin NF: chitin nanofiber
Chitosan NF: chitosan nanofiber
TEMPO: 2,2,6,6-tetramethylpiperidine 1-oxyl
DMSO: dimethyl sulfoxide

The properties of dispersion liquids obtained in Examples and Comparative Examples were measured as described below.

[Measurement of Solid Content Concentration]

The measurement of solid content concentration was performed using a moisture analyzer (MOC63u, manufactured by Shimadzu Corporation). The solid content concentration of a dispersion liquid was measured in a standard drying automatic ending mode (drying temperature: 105°C, ending condition: a moisture change rate per 30 seconds of 0.05% or less).

[Measurement of Shape (Fiber Length and Fiber Diameter)]

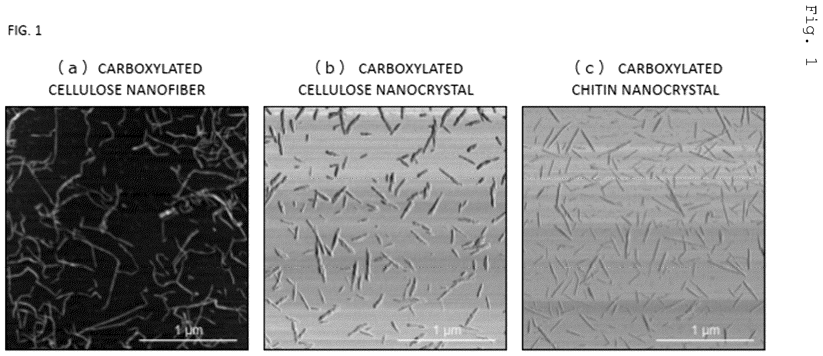

A fiber shape was measured using a scanning probe microscope. 10 μL of a dispersion liquid diluted to 0.005 wt% with ultrapure water was dropped onto a mica substrate and dried at room temperature to prepare a sample for observation. The sample was set in a scanning probe microscope (Environment Control Unit E-sweep, manufactured by Hitachi High-Tech Science Corporation), and a microcantilever SI-DF40 (manufactured by Hitachi High-Tech Science Corporation) was used to acquire fiber image in a DFM mode (FIG. 1(a), FIG. 1(b), and FIG. 1(c)).

50 fibers were randomly selected from the image, and the fiber length and fiber diameter of each of the fibers were measured with image analysis software (Gwyddion, http://gwyddion.net/) and were each expressed as a numerical range with a minimum and a maximum. In addition, the averages of the fiber lengths and the fiber diameters were determined as arithmetic averages. The maximum value for the fiber lengths of the fibers was difficult to accurately measure, and hence was shown as a rough value.

[Introduced Functional Group Content Measurement 1] (Carboxyl Group Concentration)

A carboxyl group concentration was measured by a conductometric titration method. Specifically, a nanocellulose dispersion liquid or a nanochitin dispersion liquid was adjusted to 0.3 wt%, and then adjusted to a pH of about 2.0 with a 0.1 N aqueous solution of hydrochloric acid. Through use of a potentiometric automatic titration apparatus (AT-510, manufactured by Kyoto Electronics Manufacturing Co., Ltd.), a 0.05 N aqueous solution of sodium hydroxide was dropped, and an electrical conductivity was measured until the pH reached 11. The carboxyl group concentration was determined in accordance with the following equation from a sodium hydroxide amount (V) consumed at a weak acid neutralization stage at which the change in electrical conductivity was little.

```
Carboxyl        group        concentration        (mmol/g)=V
(mL)×0.05/nanocellulose weight (g)
```

```
Carboxyl        group        concentration        (mmol/g)=V
(mL)×0.05/nanochitin weight (g)
```

[Introduced Functional Group Content Measurement 2] (Phosphoric Acid Ester Group or Sulfuric Acid Ester Group Concentration)

With reference to a related art document (Japanese Patent Application Laid-open No. 2017-25468), a phosphoric acid ester group or sulfuric acid ester group concentration was measured. Specifically, a phosphorylated CNF dispersion liquid or a sulfated CNF dispersion liquid was adjusted to 0.2 wt%, and treated with a strongly acidic ion-exchange resin. After that, a 0.1 N aqueous solution of sodium hydroxide was dropped, and an electrical conductivity was measured. The phosphoric acid ester group or sulfuric acid ester group concentration was determined in accordance with the following equation from a sodium hydroxide amount (V) consumed at a strong acid neutralization stage.

```
Phosphoric  acid  ester  group  concentration  (mmol/g)=V
(mL)×0.1/nanocellulose weight (g)
```

```
Sulfuric  acid  ester  group  concentration  (mmol/g)=V
(mL)×0.1/nanocellulose weight (g)
```

[Measurement of Viscosity]

A viscosity is measured using a rotational viscometer (HAAKE RS-6000, manufactured by Thermo Fisher Scientific K.K.). In practice, in a Rot mode (rotational viscosity measurement), each dispersion liquid was placed on a lower plate (Lower Plate TMP60) and subjected to measurement using a sensor (Cone C60/1° Ti L) (Rot time dependence measurement; mode: CR, shear rate: 100 1/s, gap: 0.052 mm, temperature: 25°C and 37°C), and an average viscosity value over 5 minutes from the start of the measurement was measured. The viscosity of pure water was 0.83 mPa·s at 25°C, and 0.70 mPa·s at 37°C.

[Production Example of CNF Having Carboxyl Group Introduced Therein]

With reference to a related art document (Japanese Patent Application Laid-open No. 2013-249448, production of cellulose fiber B6), a CNF having a carboxyl group introduced therein was produced.

Specifically, 150 mL of water, 0.25 g of sodium bromide, and 0.025 g of a TEMPO reagent were added to 2 g of softwood pulp, and a 13 wt% aqueous solution of sodium hypochlorite (co-oxidant) was added at 24.0 mmol/g to initiate a reaction. During the reaction, the pH of the dispersion liquid was kept at from 10 to 11 with dropwise addition of 0.5 N sodium hydroxide, and the reaction was performed until no change in pH was observed (the reaction time was 120 minutes) .

After the completion of the reaction, 0.1 N hydrochloric acid was added for neutralization, and purification was performed by repeating filtration and water washing to give a cellulose fiber having an oxidized microfibril surface. Solid-liquid separation was performed with a centrifuge, and then pure water was added to adjust the solid content concentration to 4 wt%.

The pH of the slurry was adjusted to 10 with a 24% aqueous solution of sodium hydroxide, and the temperature of the slurry was controlled to 30°C. Reductive treatment was performed by adding sodium borohydride at 0.2 mmol/g with respect to the cellulose fiber, followed by a 2-hour reaction. After the reaction, a 0.1 N hydrochloric acid was added for neutralization, and purification was performed by repeating filtration and water washing to give a cellulose fiber.

The cellulose fiber was diluted to 1 wt% by adding pure water thereto, and the resultant was treated once at a pressure of 100 MPa through use of a high-pressure homogenizer (manufactured by Sanwa Engineering Ltd., H11). Thus, a CNF having a carboxyl group introduced therein was produced.

[Examples 1 to 4] (Preparation of Carboxylated CNF Dispersion Liquids)

Distilled water (Otsuka Distilled Water, manufactured by Otsuka Pharmaceutical Factory, Inc.) was added to the above-mentioned CNF having a carboxyl group introduced therein so as to achieve solid content concentrations of 0.2 wt% (Example 1), 0.4 wt% (Example 2), 0.8 wt% (Example 3), and 1.2 wt% (Example 4), followed by high-speed rotary mixer (Polytron Homogenizer, manufactured by Central Scientific Commerce, Inc.) treatment to give uniform carboxylated CNF dispersion liquids. The resultant dispersion liquids were subjected to high-pressure steam sterilization treatment at 121°C for 20 minutes.

[Production Example of CNF Having Phosphoric Acid Ester Group Introduced Therein]

With reference to a related art document (Japanese Patent Application Laid-open No. 2017-25468, Comparative Example 2), a CNF having a phosphoric acid ester group introduced therein was produced.

Specifically, 30.0 g of urea, 16.6 g of sodium dihydrogen phosphate dihydrate, and 12.4 g of disodium hydrogen phosphate were dissolved in 32.8 g of pure water to prepare a phosphorylation reagent. A sheet of softwood bleached kraft pulp was treated with a cutter mill and a pin mill to provide a cotton-like fiber. After that, 30 g of the fiber was taken in terms of absolute dry weight, and uniformly sprayed with the phosphorylation reagent to give chemical-impregnated pulp.

Next, the chemical-impregnated pulp was subjected to heat treatment for 120 minutes with a blow drying machine with a damper heated to 140°C to give phosphorylated pulp. 3 g of the phosphorylated pulp was taken in terms of pulp mass, 300 mL of ion-exchanged water was added, and the mixture was stirred to be uniformly dispersed, followed by filtration dewatering to give a dewatered sheet. This step was repeated twice. Then, the resultant dewatered sheet was diluted with 300 mL of ion-exchanged water. While the resultant was stirred, 1 N sodium hydroxide was added dropwise, and 300 mL of ion-exchanged water was added to give a pulp slurry having a pH of from 12 to 13. The pulp slurry was dewatered to give a dewatered sheet, 300 mL of ion-exchanged water was then added, and the mixture was stirred to be uniformly dispersed, followed by filtration dewatering to give a dewatered sheet. This step was repeated twice.

Ion-exchanged water was added to the dewatered sheet of the phosphorylated pulp obtained after washing and dewatering, and the mixture was stirred to provide a 0.5 wt% slurry. The slurry was treated at 21,500 rotations/min for 30 minutes using a fibrillation treatment apparatus (manufactured by M Technique Co., Ltd., CLEARMIX-2.2S) . Thus, a CNF having a phosphoric acid ester group introduced therein was produced.

[Examples 5 and 6] (Preparation of Phosphorylated CNF Dispersion Liquids)

Distilled water was added to the above-mentioned CNF having a phosphoric acid ester group introduced therein so as to achieve solid content concentrations of 0.8 wt% (Example 5) and 1.2 wt% (Example 6), followed by high-speed rotary mixer treatment to give uniform phosphorylated CNF dispersion liquids. The resultant dispersion liquids were subjected to high-pressure steam sterilization treatment at 121°C for 20 minutes.

[Production Example of CNF Having Sulfuric Acid Ester Group Introduced Therein]

With reference to a related art document (WO 2018/131721 A1, Example 14), a CNF having a sulfuric acid ester group introduced therein was produced.
Specifically, 18 g of DMSO, 2 g of acetic anhydride (concentration in fibrillation solution: 9.8 wt%), 0.5 g of sulfuric acid (concentration in fibrillation solution: 2.4 wt%) were placed in a 50 mL sample bottle, and the contents were thoroughly stirred to prepare a fibrillation solution. To the fibrillation solution, 0.6 g of cellulose pulp was added, and the mixture was stirred at room temperature (23°C) for 80 minutes. Next, 400 mL of a 0.75 wt% aqueous solution of sodium hydrogen carbonate was added, followed by mixing at room temperature (23°C) for 10 minutes. After that, a supernatant was removed by centrifugation. 400 mL of pure water was added to the remaining precipitate (cellulose fiber), and the mixture was stirred to be uniformly dispersed, followed by the removal of a supernatant by centrifugation. The same operation was repeated 4 times.
The precipitate obtained by the centrifugation was diluted by adding pure water thereto until the total weight became 150 g. The resultant was stirred for 3 minutes using a high-speed rotary mixer (Polytron Homogenizer, manufactured by Central Scientific Commerce, Inc.). Thus, a CNF having a sulfuric acid ester group introduced therein was produced.

[Example 7] (Preparation of Sulfated CNF Dispersion Liquid)

Distilled water was added to the above-mentioned CNF having a sulfuric acid ester group introduced therein so as to achieve a solid content concentration of 0.8 wt% (Example 7), followed by high-speed rotary mixer treatment to give a uniform sulfated CNF dispersion liquid.

[Comparative Example 1] (Preparation of Unmodified CNF Dispersion Liquid)

Commercially available BiNFi-s (trademark), WMa-10002 (manufactured by Sugino Machine Limited, solid content concentration: 2.0 wt%) was used as an unmodified CNF free of chemical modification on its cellulose microfibril surface. Distilled water was added thereto so as to achieve a solid content concentration of 0.8 wt% (Comparative Example 1), followed by high-speed rotary mixer treatment to give an unmodified CNF dispersion liquid. The resultant dispersion liquid was subjected to high-pressure steam sterilization treatment at 121°C for 20 minutes.

[Comparative Examples 2 and 3] (Preparation of CMC Solutions)

Commercially available sodium carboxymethyl cellulose (CELLOGEN AG GUM M, official, degree of etherification: 0.82, manufactured by DKS Co. Ltd.) was used as cellulose having no microfibrils. The cellulose was dissolved in distilled water so as to achieve solid content concentrations of 0.8 wt% (Comparative Example 2) and 1.5 wt% (Comparative Example 3), followed by high-pressure steam sterilization treatment at 121°C for 20 minutes.

[Examples 8 to 13] (Preparation of Carboxylated CNC Dispersion Liquids)

A commercially available carboxylated CNC (manufactured by Cellulose Lab, TEMPO-oxidized product, functional group concentration: 1.2 mmol/g or 2.0 mmol/g, solid content concentration: 2.0 wt%) was used as a CNC having a carboxyl group introduced at the 6-position of glucose on its cellulose microfibril surface. Distilled water was added thereto so as to achieve solid content concentrations of 0.1 wt% (Example 8), 0.5 wt% (Example 9), 1.0 wt% (Example 10), 1.5 wt% (Example 11), 2.0 wt% (Example 12), and 1.2 wt% (Example 13), followed by high-speed rotary mixer treatment to give uniform carboxylated CNC dispersion liquids. The resultant dispersion liquids were subjected to high-pressure steam sterilization treatment at 121°C for 20 minutes.

[Production Example of CNC Having Carboxyl Groups Introduced Therein]

With reference to a related art document (WO 2018/230354 A1, ACS Sustainable Chem Eng 2020, 8, 17800-17806), a CNC having carboxyl groups introduced at the 2-position and 3-position of glucose on its cellulose microfibril surface was produced.

Specifically, water, 6 M hydrochloric acid, an unmodified CNC (solid content: 1 g) were added to 42.8 g of sodium hypochlorite pentahydrate (manufactured by FUJIFILM Wako Pure Chemical Corporation), and the mixture was stirred to provide an aqueous solution having a concentration of 22% and a pH of 11.0. While the aqueous solution was kept at a temperature of 30°C, 5 M sodium hydroxide was added to maintain the pH at 11.0, and the whole was stirred with a stirrer for 2 hours. Sodium sulfite was added to the resultant liquid until its oxidation-reduction potential became -100 mV or less. Thus, residual sodium hypochlorite was reduced, and the reaction was completed. The resultant was placed in a dialysis tube (material: regenerated cellulose, molecular weight cutoff: 3,500, manufactured by Fisher Scientific), and dialyzed for 1 week. The suspension liquid was subjected to ultrasonic crushing with an ultrasonic homogenizer (LUH300, manufactured by Yamato Scientific Co., Ltd.) by performing interval operation (ON for 1 minute→OFF for 1 minute) for 3 minutes in a constant amplitude control mode (output amplitude preset value: 55%). The suspension liquid after the crushing treatment was centrifuged, and the supernatant was collected. Thus, a CNC having carboxyl groups introduced therein was produced.

[Example 14] (Preparation of Carboxylated CNC Dispersion Liquid)

Distilled water (Otsuka Distilled Water, manufactured by Otsuka Pharmaceutical Factory, Inc.) was added to the above-mentioned CNC having carboxyl groups introduced therein so as to achieve a solid content concentration of 1.0 wt% (Example 14), to thereby give a carboxylated CNC dispersion liquid. The resultant dispersion liquid was subjected to high-pressure steam sterilization treatment at 121°C for 20 minutes.

[Production Example of CNC Having Sulfuric Acid Ester Group Introduced Therein]

With reference to a related art document (Cellulose Commun, 2016, 23, 193-199), a CNC having a sulfuric acid ester group introduced therein was produced.

Specifically, 10 g of wood-derived microcrystalline cellulose (Nacalai Tesque, Inc.) was suspended in 100 mL of 65% sulfuric acid, and the whole was stirred at 45°C for 60 minutes. 200 mL of distilled water was added to quench the reaction. The reaction liquid was centrifuged, and the supernatant was discarded by decantation. An appropriate amount of distilled water was added to the precipitate, and the whole was vigorously shaken to completely suspended the precipitate, followed by centrifugation again. This washing step was repeated while the supernatant after the centrifugation was transparent. When the supernatant was brought into a turbid state after the centrifugation, the supernatant was collected. The collecting step was continued while the supernatant was turbid, and the collection was ended when the turbidity disappeared. The collected CNC suspension liquid was placed in a dialysis membrane (MWCO14000), and dialyzed until the external dialysis solution became neutral. While being placed in the dialysis membrane, the CNC suspension liquid was dipped in a polyethylene glycol (molecular weight: 20,000) solution having a concentration of 10%, and was concentrated to a solid content concentration of about 2%.

[Example 15] (Preparation of Sulfated CNC Dispersion Liquid)

Distilled water (Otsuka Distilled Water, manufactured by Otsuka Pharmaceutical Factory, Inc.) was added to the above-mentioned CNC having a sulfuric acid ester group introduced therein so as to achieve a solid content concentration of 1.0 wt% (Example 15), to thereby give a sulfated CNC dispersion liquid. The resultant dispersion liquid was subjected to high-pressure steam sterilization treatment at 121°C for 20 minutes.

[Production Example of CNC Having Carboxymethyl Group Introduced Therein]

With reference to a related art document (LWT-Food Science and Technology, 2017, 86, 318-326), a CNC having a carboxymethyl group introduced therein was produced.

Specifically, 1 g of CNC powder (manufactured by Cellulose Lab) was suspended in 100 mL of isopropanol, and the whole was stirred. 1 mL of a 12 N aqueous solution of sodium hydroxide was added dropwise, and the stirring was continued. 1.42 g of chloroacetic acid was dissolved in 15 mL of isopropanol, the resultant solution was added dropwise, and the whole was stirred at 50°C for 50 minutes. 1 mL of a 12 N aqueous solution of sodium hydroxide was added dropwise, and the whole was further stirred at 60°C for 1 hour. 0.5 mL of a 90% aqueous solution of acetic acid was added dropwise to neutrality. The reaction liquid was left to stand still until its temperature was reduced to room temperature. The resultant was filtered through a Kiriyama funnel, and the residue was washed twice with 75 mL of 80% methanol and then washed once with 50 mL of methanol. The resultant was dried at 45°C for 24 hours to give a powdery CNC having a carboxymethyl group introduced therein.

[Example 16] (Preparation of Carboxymethylated CNC Dispersion Liquid)

The above-mentioned CNC having a carboxymethyl group introduced therein was dissolved in distilled water (Otsuka Distilled Water, manufactured by Otsuka Pharmaceutical Factory, Inc.) so as to achieve a solid content concentration of 1.0 wt% (Example 16), followed by high-pressure steam sterilization treatment at 121°C for 20 minutes.

[Comparative Example 4] (Preparation of Unmodified CNC Dispersion Liquid)

A commercially available CNC (manufactured by Cellulose Lab, desulfated product, solid content concentration: 2.0 wt%) was used as an unmodified CNC free of chemical modification on its cellulose microfibril surface. Distilled water was added thereto so as to achieve a solid content concentration of 1.5 wt% (Comparative Example 4), followed by high-speed rotary mixer treatment to give a uniform unmodified CNC dispersion liquid. The resultant dispersion liquid was subjected to high-pressure steam sterilization treatment at 121°C for 20 minutes.

[Comparative Example 5] (Preparation of Quaternary Ammoniated CNC Dispersion Liquid)

A commercially available quaternary ammoniated CNC (manufactured by Cellulose Lab, solid content concentration: 1.5 wt%) (Comparative Example 5) was used as a CNC having a quaternary ammonium group introduced on its cellulose microfibril surface. High-speed rotary mixer treatment was performed, followed by high-pressure steam sterilization treatment at 121°C for 20 minutes.

[Production Example of Chitin NC Having Carboxyl Group Introduced Therein]

With reference to the method of Fan et al. (Biomacromolecules 2008, 9, 192-198), a carboxylated chitin NC was produced.
Specifically, 300 mL of water, 0.3 g of sodium bromide, and 0.048 g of a TEMPO reagent were added to 3 g of chitin powder (manufactured by FUJIFILM Wako Pure Chemical Corporation), and 10.1 g of sodium hypochlorite pentahydrate (effective chlorine: 39% or more) was added to initiate a reaction at room temperature. During the reaction, the pH of the dispersion liquid was kept at 10 with dropwise addition of a 0.5 N aqueous solution of sodium hydroxide, and the reaction was performed for 45 minutes, followed by the addition of 10 mL of ethanol (99.5) to quench the oxidation reaction. The pH of the reaction liquid was adjusted to near 7.0 with a 0.5 N aqueous solution of sodium hydroxide, and then a supernatant was removed by centrifugation. Ultrapure water was added to the remaining precipitate, and the mixture was stirred to be uniformly dispersed, followed by the removal of a supernatant by centrifugation. The same operation was repeated 3 times.
The precipitate obtained by centrifugation was suspended by adding 50 mL of ultrapure water thereto, and the resultant was placed in a dialysis tube (material: regenerated cellulose, molecular weight cutoff: 3,500, manufactured by Fisher Scientific) and dialyzed for 5 days. The suspension liquid was subjected to ultrasonic crushing with an ultrasonic homogenizer (LUH300, manufactured by Yamato Scientific Co., Ltd.) by performing interval operation (ON for 1 minute, OFF for 1 minute) for 60 minutes in a constant amplitude control mode (output amplitude preset value: 55%). The suspension liquid after the crushing treatment was centrifuged, and the supernatant was collected. Thus, a chitin NC having a carboxyl group introduced therein was produced.

[Example 17] (Preparation of Carboxylated Chitin NC)

Distilled water was added to the above-mentioned chitin NC having a carboxyl group introduced therein so as to achieve a solid content concentration of 1.0 wt% (Example 17), followed by high-speed rotary mixer treatment to give a uniform carboxylated chitin NC dispersion liquid.

[Comparative Example 6] (Preparation of Unmodified Chitin NF Dispersion Liquid)

A commercially available chitin NF (BiNFi-s (trademark), SFo-20002, manufactured by Sugino Machine Limited, solid content concentration: 2.0 wt%) was used as an unmodified chitin NF free of chemical modification on its chitin microfibril surface. Distilled water was added thereto so as to achieve a solid content concentration of 1.0 wt% (Comparative Example 6), followed by high-speed rotary mixer treatment to give a uniform unmodified chitin NF dispersion liquid. The resultant dispersion liquid was subjected to high-pressure steam sterilization treatment at 121°C for 20 minutes.

[Comparative Example 7] (Preparation of Chitosan NF Dispersion Liquid)

A commercially available chitosan NF (BiNFi-s (trademark), EFo-20002, manufactured by Sugino Machine Limited, solid content concentration: 2.0 wt%) was used as a chitosan NF free of chemical modification on its chitosan microfibril surface. Distilled water was added thereto so as to achieve a solid content concentration of 1.0 wt% (Comparative Example 7), followed by high-speed rotary mixer treatment to give a uniform unmodified chitosan NF dispersion liquid. The resultant dispersion liquid was subjected to high-pressure steam sterilization treatment at 121°C for 20 minutes.
The measurement results of the properties (shape, functional group, and dispersion liquid viscosity) of the dispersion liquids of Examples 1 to 17 and Comparative Examples 1 to 7 are shown in Table 1.

Table 1

| No. | Sample name | Shape | | | Functional group | | | Dispersion liquid viscosity | | |
| | | Classification | Fiberlength (nm) | Fiber diameter (nm) | Polarity | Modifying group | Concentr ation (mmol/g) | Concent ration (wt%) | 25°C | 37°C |
| | | | | | | | | | (mPa·s) | |
| Example 1 | Carboxylated CNF | Fiber form | 500 to 5,000 | 3 to 4 | Anion | Carboxyl group | 2.0 | 0.2 | 45.3 | 39.8 |
| Example 2 | | | | | | | | 0.4 | 117 | 123 |
| Example 3 | | | | | | | | 0.8 | 734 | 684 |
| Example 4 | | | | | | | | 1.2 | 1, 17 6 | 1, 100 |
| Example 5 | Phosphorylated CNF | | 500 to 5,000 | 3 to 4 | Anion | Phosphoric acid ester group | 1.3 | 0.8 | 807 | 680 |
| Example 6 | | | | | | | | 1.2 | 1, 75 9 | 1, 726 |
| Example 7 | Sulfated CNF | | 500 to 5,000 | 3 to 4 | Anion | Sulfuric acid ester group | 1.45 | 0.8 | 907 | 963 |
| Comparative Example 1 | Unmodified CNF | | 500 to 5,000 | 10 to 50 | Nonion | - | - | 0.8 | 45.5 | 73.3 |
| Comparative Example 2 | CMC | Molecular chain | - | - | Anion | Carboxylmethyl group | 3.9* | 0.8 | 41.1 | 30.2 |
| Comparative Example 3 | | | | | | | | 1.5 | 148 | 107 |

EP 4 159 251 A1

(continued)

| No. | Sample name | Shape | | | Functional group | | | Dispersion liquid viscosity | | |
|---|---|---|---|---|---|---|---|---|---|---|
| | | Classification | Fiberlength (nm) | Fiber diameter (nm) | Polarity | Modifying group | Concentr ation (mmol/g) | Concent ration (wt%) | 25°C | 37°C |
| | | | | | | | | | (mPa·s) | |
| Example 8 | Carboxylated CNC | Crystal form | 140 to 200 | 5 to 20 | Anion | Carboxyl group | 1.2 | 0.1 | 0.91 | 0.74 |
| Example 9 | | | | | | | | 0.5 | 1.71 | 0.99 |
| Example 10 | | | | | | | | 1.0 | 2.34 | 1.38 |
| Example 11 | | | | | | | | 1.5 | 2.43 | 2.00 |
| Example 12 | | | | | | | | 2.0 | 3.38 | 2.77 |
| Example 13 | | | | | | | 2.0 | 1.2 | 36.9 | 36.5 |
| Example 14 | | | | | | | 0.33 | 1.0 | 1.45 | 1.41 |
| Example 15 | Sulfated CNC | | 140 to 200 | 5 to 20 | Anion | Sulfuric acid ester group | 0.19 | 1.0 | 1.34 | 1.26 |
| Example 16 | Carboxymethylated CNC | | 140 to 200 | 5 to 20 | Anion | Carboxymethyl group | 3.31 | 1.0 | 1.33 | 1.13 |
| Comparative Example 4 | Unmodified CNC | | 140 to 200 | 5 to 20 | Nonion | - | - | 1.5 | 2. 97 | 2.51 |
| Comparative Example 5 | Quaternary ammoniated CNC | | 140 to 200 | 5 to 20 | Cation | Quaternary ammonium group | 0.15 | 1.5 | 41.7 | 36.9 |
| Example 17 | Carboxylated chitin NC | | 140 to 200 | 3 to 4 | Anion | Carboxyl group | 1.4 | 1.0 | 1.65 | 1.27 |
| Comparative Example 6 | Unmodified chitin NF | Fiber form | 500 to 5,000 | 10 to 50 | Nonion | - | - | 1.0 | 108 | 109 |
| Comparative Example 7 | Chitosan NF | Fiber form | 500 to 5,000 | 20 to 50 | Cation | - | - | 1.0 | 12.1 | 24.2 |

*A concentration (mmol/g) converted from the degree of etherification (0.82) is shown.

18

[Example 18] (Adhesion Preventing Effect of Carboxylated CNF Dispersion Liquid with Mouse Adhesion Model - Comparison to Unmodified CNF Dispersion Liquid and CMC Solution)

(Test Method)

6- to 7-week-old male BALB/cA Jcl mice (CLEA Japan, Inc.) were used. The abdomen was opened under anesthesia with a ketamine/xylazine cocktail, and then the cecum was exposed. One site on the opposite side of the cecum to the mesentery was ablated for 1 second at an output of 5 W (SurgiStat, Covidien Japan Inc.) through use of a bipolar coagulation forceps (E4052-CT, Covidien Japan Inc.). After that, the carboxylated CNF dispersion liquid (Example 3), the unmodified CNF dispersion liquid (Comparative Example 1), or the CMC solution (Comparative Example 2) was administered at 0.25 mL/body to the inside of the peritoneal cavity by dropping, and the abdomen was closed. A control group was administered an equal amount of saline. For this test method, reference was made to the method of Kosaka et al. (Nature Medicine Letters 2008, Vol.14, No.4, 437-441). On the 7th day after the surgery, under a state in which the test groups were kept secret, adhesion at the cecum ablation portion and other organs were evaluated with scores on the following 6-point scale. For each group, the mean±standard error of the adhesion scores was calculated.

    Score 0: No adhesion
    Score 1: One thin filmy adhesion
    Score 2: More than one thin adhesion
    Score 3: Thick adhesion with focal point
    Score 4: Thick adhesion with plantar attachment or more than one thick adhesion with focal point
    Score 5: Thick adhesion with plantar attachment or more than one thick adhesion with focal point

(Test Results)

The results of the adhesion evaluation are shown in FIG. 2. The carboxylated CNF dispersion liquid clearly reduced the adhesion score as compared to the control group. Meanwhile, the unmodified CNF dispersion liquid and the CMC solution did not reduce the adhesion score.

It was found from those results that the cellulose in the dispersion liquid was in a fiber form having a microfibril structure (assembly of cellulose molecular chains), and that the modification of the surface thereof with a carboxyl group provided an adhesion preventing action.

[Example 19] (Adhesion Preventing Effect of Carboxylated CNC Dispersion Liquid with Mouse Adhesion Model - Comparison to Unmodified CNC Dispersion Liquid, Quaternary Ammoniated CNC Dispersion Liquid, and CMC Solution)

(Test Method)

A test was performed by the same method and under the same conditions as in Example 18. The carboxylated CNC dispersion liquid (Example 11), the unmodified CNC dispersion liquid (Comparative Example 4), the quaternary ammoniated CNC dispersion liquid (Comparative Example 5), or the CMC solution (Comparative Example 3) was administered at 0.25 mL/body. A control group was administered an equal amount of saline.

(Test Results)

The results of the adhesion evaluation are shown in FIG. 3. The carboxylated CNC dispersion liquid clearly reduced the adhesion score as compared to the control group. Meanwhile, the unmodified CNC dispersion liquid, the quaternary ammoniated CNC dispersion liquid, and the CMC solution did not reduce the adhesion score.

It was found from those results that the cellulose in the dispersion liquid was in a crystal form having a microfibril structure, and that the modification of the surface thereof with a carboxyl group provided an adhesion preventing action. Meanwhile, the quaternary ammoniated CNC dispersion liquid did not show an adhesion preventing effect, suggesting that it was important that the modifying functional group on the microfibril surface be not cationic, but anionic.

[Example 20] (Adhesion Preventing Effect of Anionized CNF Dispersion Liquid with Rat Small Intestine Adhesion Model - Comparison to Unmodified CNF Dispersion Liquid)

(Test Method)

7- to 8-week-old male Crl:CD(SD) rats (Charles River Laboratories Japan, Inc.) were used. The abdomen was opened under anesthesia with a ketamine/xylazine cocktail, and periepididymal fat was resected. The ileum was exposed to the outside of the body, and the small intestine in the range of 10 cm from the cecum was rubbed with dry gauze wrapped around a finger to such an extent that hemorrhage was observed. The organ was returned to the inside of the peritoneal cavity, and the inside of the peritoneal cavity was washed with 10 mL of saline. The residual liquid after the washing was removed by suction as much as possible. After that, the carboxylated CNF dispersion liquid (Example 3), the phosphorylated CNF dispersion liquid (Example 5), the sulfated CNF dispersion liquid (Example 7), or the unmodified CNF dispersion liquid (Comparative Example 1) was administered at 2 mL/body to the inside of the peritoneal cavity by dropping, and the abdomen was closed. A control group was administered an equal amount of saline. For this test method, reference was made to the method of Yamauchi et al. (Japanese Patent Application Laid-open No. Hei 7-101866) . On the 7th day after the surgery, under a state in which the test groups were kept secret, the length of adhesion formed over a wide range inside the peritoneal cavity including the bowel was measured. For each group, the mean$\pm$standard error of the lengths (mm) of adhesion was calculated.

(Test Results)

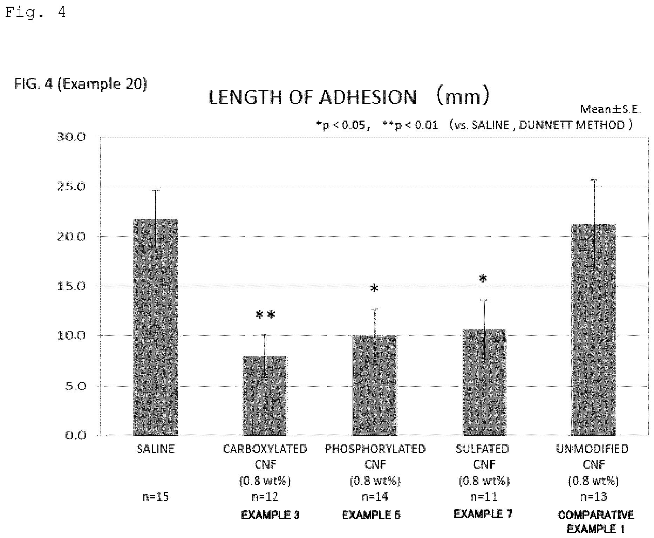

The results of the adhesion evaluation are shown in FIG. 4. The carboxylated CNF dispersion liquid, the phosphorylated CNF dispersion liquid, and the sulfated CNF dispersion liquid each clearly reduced the length of adhesion as compared to the control group. Meanwhile, the unmodified CNF dispersion liquid did not reduce the length of adhesion.
It was found from those results that the chemical modification of the microfibril surface of the cellulose nanofiber provided an adhesion preventing action in the case of substitution with an anionic functional group, such as a phosphoric acid ester group or a sulfuric acid ester group, not limited to a carboxyl group.

[Example 21] (Adhesion Preventing Effects of Various Anionized CNC Dispersion Liquids with Rat Small Intestine Adhesion Model - Comparison to Saline)

(Test Method)

A test was performed by the same method and under the same conditions as in Example 20. The carboxylated CNC dispersion liquid (Example 14, obtained by modifying the hydroxy groups at the 2-position and the 3-position of glucose into carboxyl groups), the sulfated CNC dispersion liquid (Example 15), or the carboxymethylated CNC dispersion liquid (Example 16) was administered at 2 mL/body. Control groups were each administered an equal amount of saline.

(Test Results)

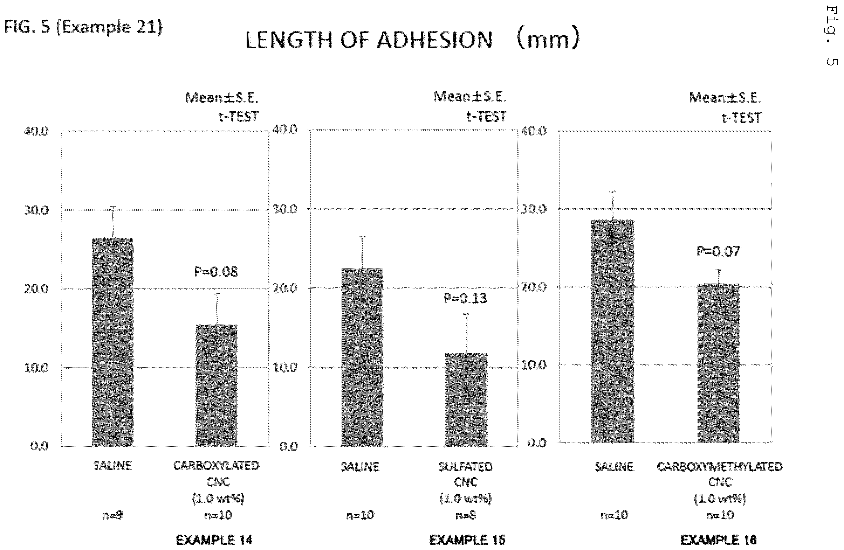

The results of the adhesion evaluation are shown in FIG. 5. The carboxylated CNC dispersion liquid, the sulfated CNC dispersion liquid, and the carboxymethylated CNC dispersion liquid each reduced the length of adhesion as compared to the control group.
It was found from those results that the chemical modification of the microfibril surface of the cellulose nanocrystal provided an adhesion preventing action in the case of substitution with an anionic functional group, such as a sulfuric acid ester group or a carboxymethyl group, not limited to a carboxyl group. Further, the CNC obtained by modifying the hydroxy groups at the 2-position and the 3-position of glucose into carboxyl groups (Example 14), and as shown in Example 23, the CNC obtained by modifying the hydroxy group at the 6-position of glucose into a carboxyl group (Example 10) each reduced the length of adhesion, and hence it was found that the adhesion preventing action was not selective in terms of the position modified with a carboxyl group.

[Example 22] (Adhesion Preventing Effect of Carboxylated CNF Dispersion Liquid with Rat Small Intestine Adhesion Model - Concentration Dependence)

(Test Method)

A test was performed by the same method and under the same conditions as in Example 20. The 0.2 wt% (Example 1), 0.4 wt% (Example 2), and 0.8 wt% (Example 3) carboxylated CNF dispersion liquids were administered at 2 mL/body. A control group was administered an equal amount of saline.

(Test Results)

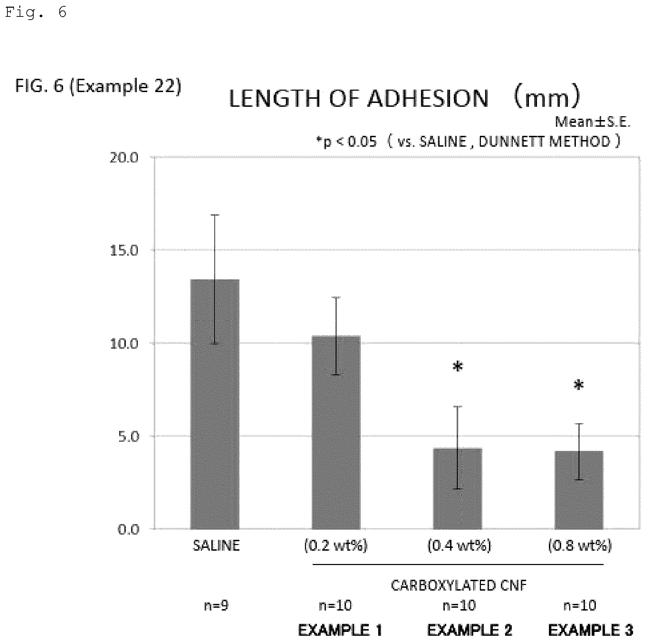

The results of the adhesion evaluation are shown in FIG. 6. The length of adhesion was reduced at concentrations of 0.2 wt%, 0.4 wt%, and 0.8 wt% as compared to the control group. Further, the length of adhesion was significantly reduced at concentrations of 0.4 wt% and 0.8 wt%.

[Example 23] (Adhesion Preventing Effect of Carboxylated CNC Dispersion Liquid with Rat Small Intestine Adhesion Model - Concentration Dependence)

(Test Method)

A test was performed by the same method and under the same conditions as in Example 20. The 0.1 wt% (Example 8), 0.5 wt% (Example 9), 1.0 wt% (Example 10), 1.5 wt% (Example 11), and 2.0 wt% (Example 12) carboxylated CNC dispersion liquids were administered at 2 mL/body. A control group was administered an equal amount of saline.

(Test Results)

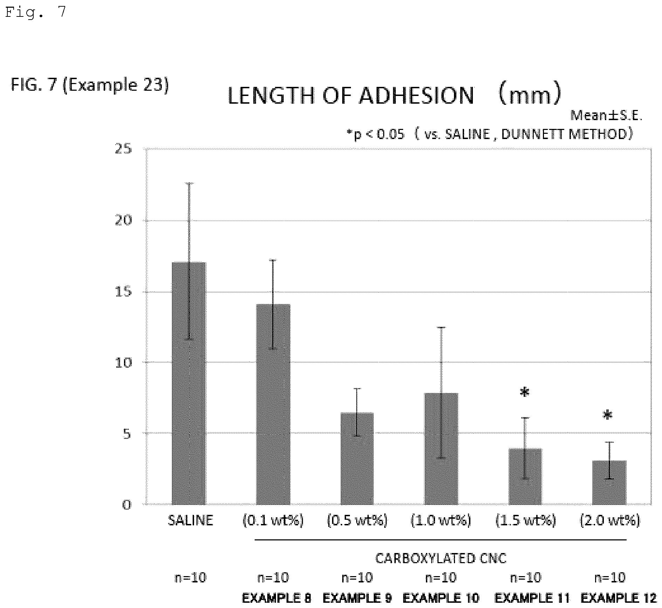

The results of the adhesion evaluation are shown in FIG. 7. The length of adhesion was reduced at all concentrations from 0.1 wt% to 2.0 wt% as compared to the control group. Further, the length of adhesion was significantly reduced at 1.5 wt% and 2.0 wt%.

[Example 24] (Comparison between Adhesion Preventing Effects of Carboxylated CNF Dispersion Liquid and Carboxylated CNC Dispersion Liquid with Rat Small Intestine Adhesion Model)

(Test Method)

A test was performed by the same method and under the same conditions as in Example 20 . The 1.2 wt% carboxylated CNF dispersion liquid (Example 4) and the 1.2 wt% carboxylated CNC (Example 13) dispersion liquid were administered at 2 mL/body. A control group was administered an equal amount of saline.

(Test Results)

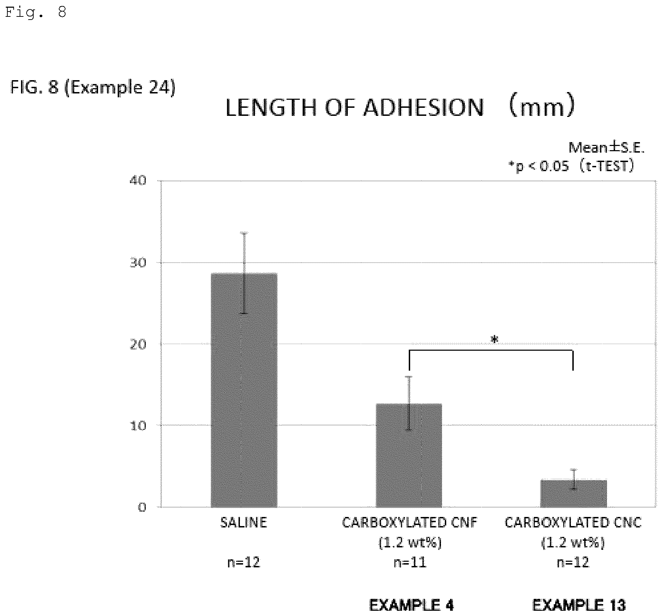

The results of the adhesion evaluation are shown in FIG. 8. The carboxylated CNF dispersion liquid and the carboxylated CNC dispersion liquid each reduced the length of adhesion as compared to the control group, and the carboxylated CNC dispersion liquid significantly reduced the length of adhesion as compared to the carboxylated CNF dispersion liquid.
It was found from those results that the carboxylated CNC was preferred over the carboxylated CNF from the viewpoint of further improving the adhesion preventing effect.

[Example 25] (Adhesion Preventing Effect of Carboxylated Chitin NC Dispersion Liquid with Rat Small Intestine Adhesion Model - Comparison to Unmodified Chitin NF Dispersion Liquid and Chitosan NF Dispersion Liquid)

(Test Method)

A test was performed by the same method and under the same conditions as in Example 20. The carboxylated chitin NC dispersion liquid (Example 17), the unmodified chitin NF dispersion liquid (Comparative Example 6), or the chitosan NF dispersion liquid (Comparative Example 7) was administered at 2 mL/body. A control group was administered an equal amount of saline.

(Test Results)

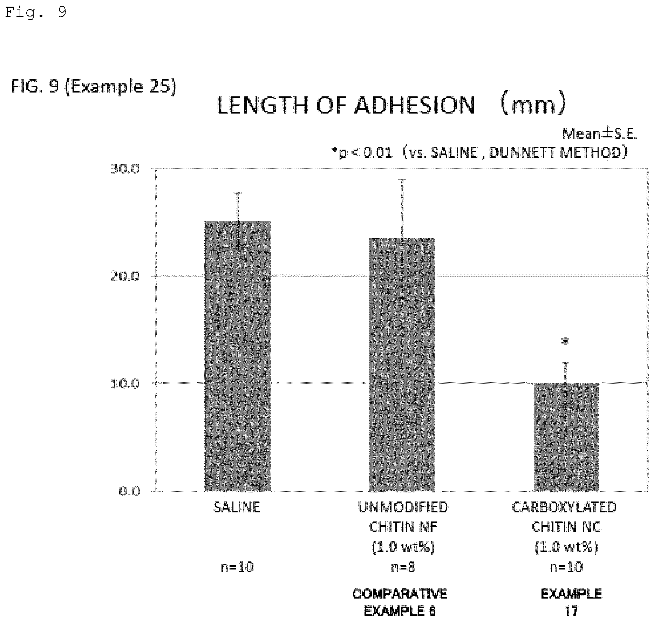

The results of the adhesion evaluation are shown in FIG. 9. The carboxylated chitin NC dispersion liquid clearly reduced the length of adhesion as compared to the control group. Meanwhile, the unmodified chitin NF dispersion liquid did not reduce the length of adhesion. The cationic chitosan NF dispersion liquid showed clear aggravation of adhesion (In 6 out of 10 cases, massive adhesion in which many organs were entangled was formed, and hence the length of adhesion was not able to be accurately measured. Accordingly, it was impossible to determine the mean of the lengths of adhesion.).
It was found from those results that, as with cellulose, the modification of the microfibril surface of chitin with a carboxyl group provided an adhesion preventing action.

[Example 26] (Adhesion Preventing Effect of Anionized CNF Dispersion Liquid with Rabbit Achilles Tendon Adhesion Model)

(Test Method)

11-week-old male Kbl:JW rabbits (Kitayama Labes Co., Ltd.) were used. The skin around the Achilles tendon (soleus muscle tendon) was shaven under anesthesia with a ketamine/xylazine cocktail, and then the knee joint and the ankle joint were fixed at 90° and 180°, respectively, through use of special braces and gypsum (3M Japan Limited). The animals were fixed in prone position, and the skin around the ankle was sufficiently disinfected by being alternately cleaning with povidone iodine (Meiji Seika Pharma Co., Ltd.) and ethanol for disinfection (FUJIFILM Wako Pure Chemical Corporation). A 2.5 cm incision was made into the skin of the treated part, and 2 cm of the surrounding tissue was resected to isolate the soleus muscle tendon. The tendon was cut with a scalpel, followed by suturing by simple ligation and the Modified Kessler method using a 5-0 suture. After it had been recognized that there was no bleeding, the site of surgery was washed with saline. The carboxylated CNF dispersion liquid (Example 4) or the phosphorylated CNF dispersion liquid (Example 6) was administered at 0.30 mL/body to the site of surgery by dropping, and the incision was closed. A control group was administered an equal amount of saline. The skin was sutured with a 3-0 nylon thread. Surgical cotton (Curelette, Kawamoto Corporation) was placed on the closed incision site, which was further covered with a bandage for cast padding (Oltex, Muranaka Medical Instruments Co., Ltd.). The area from the thigh to the toes was fixed with gypsum, and further, the gypsum was protected with an elastic bandage (ELASTPORE, Nichiban Co., Ltd.). On the 14th day after the surgery, under a state in which the test groups were kept secret, the 2 cm treated area of the soleus muscle tendon was divided into five areas, and adhesion in each of the areas was evaluated with a score on the following 5-point scale. The total for the five areas was adopted as an individual representative value, and the mean±standard error of the adhesion scores was calculated for each group.

Score 0: No adhesion
Score 1: Easy and blunt dissection is possible.
Score 2: Blunt dissection is possible.
Score 3: Sharp dissection is partly required.
Score 4: Sharp dissection is entirely required.

(Test Results)

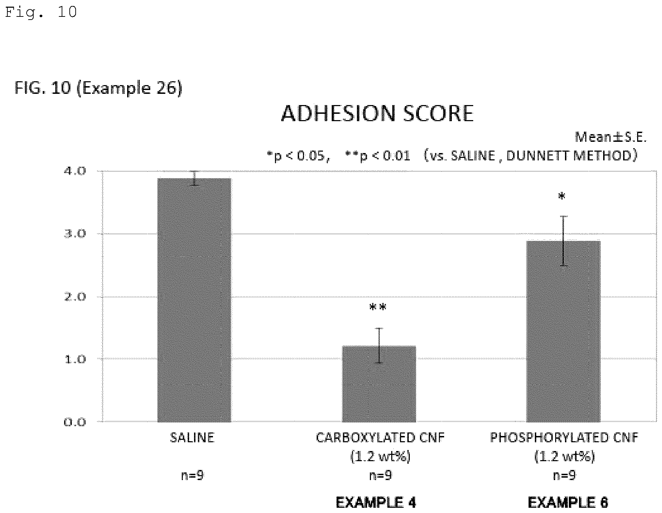

The results of the adhesion evaluation are shown in FIG. 10. The carboxylated CNF dispersion liquid and the phosphorylated CNF dispersion liquid reduced the adhesion score as compared to the control group.
It was found from those results that the anionized CNF also exhibited an adhesion preventing action on the tendon of a limb as well without being limited to the prevention of adhesion of an abdominal organ.
The adhesion preventing agent according to at least one embodiment of the present invention can effectively prevent adhesion by being applied to a site where adhesion is to be prevented.

**Claims**

1. An adhesion preventing agent, comprising as an active ingredient at least one anionized nanomaterial selected from the group consisting of anionized nanocellulose and anionized nanochitin.

2. The adhesion preventing agent according to claim 1, wherein the anionized nanomaterial is the anionized nanocellulose.

3. The adhesion preventing agent according to claim 2, wherein the anionized nanocellulose is at least one member selected from the group consisting of an anionized cellulose nanofiber and an anionized cellulose nanocrystal.

4. The adhesion preventing agent according to claim 2 or 3, wherein an anionic group contained in the anionized nanocellulose is at least one member selected from the group consisting of a carboxyl group, a carboxymethyl group, a phosphoric acid ester group and a sulfuric acid ester group.

5. The adhesion preventing agent according to claim 1, wherein the anionized nanomaterial is the anionized nanochitin.

6. The adhesion preventing agent according to claim 5, wherein the anionized nanochitin is at least one member selected from the group consisting of an anionized chitin nanofiber and an anionized chitin nanocrystal.

7. The adhesion preventing agent according to claim 5 or 6, wherein an anionic group contained in the anionized nanochitin is at least one member selected from the group consisting of a carboxyl group, a carboxymethyl group, a phosphoric acid ester group and a sulfuric acid ester group.

8. The adhesion preventing agent according to any one of claims 1 to 7, further comprising a liquid medium, wherein the anionized nanomaterial is dispersed in the liquid medium.

9. The adhesion preventing agent according to claim 8, wherein the liquid medium is a medium containing water.

10. The adhesion preventing agent according to claim 8 or 9, wherein a concentration of the anionized nanomaterial in the adhesion preventing agent is from 0.1 wt% to 10 wt%.

11. The adhesion preventing agent according to any one of claims 8 to 10, wherein the adhesion preventing agent has a viscosity of from 0.5 mPa·s to 2,000 mPa·s at 37°C.

12. The adhesion preventing agent according to any one of claims 8 to 11, wherein a content of the anionized nanomaterial in a total solid content in the adhesion preventing agent is 25 wt% or more.

13. The adhesion preventing agent according to any one of claims 1 to 12, wherein the adhesion preventing agent is for use by application to one of an organ and a tissue inside a peritoneal cavity.

14. At least one anionized nanomaterial selected from the group consisting of anionized nanocellulose and anionized nanochitin, for use in preventing adhesion of one of an organ and a tissue inside a peritoneal cavity.

15. A use of at least one anionized nanomaterial selected from the group consisting of anionized nanocellulose and anionized nanochitin, for producing an adhesion preventing agent.

16. At least one anionized nanomaterial selected from the group consisting of anionized nanocellulose and anionized nanochitin, for use as an adhesion preventing agent.

17. An adhesion preventing method, comprising applying the adhesion preventing agent of any one of claims 1 to 13 to one of an organ and a tissue inside a peritoneal cavity.

18. A method of producing an adhesion preventing agent, comprising mixing as an active ingredient at least one anionized nanomaterial selected from the group consisting of anionized nanocellulose and anionized nanochitin with a liquid medium.

Fig. 1

FIG. 1

(a) CARBOXYLATED CELLULOSE NANOFIBER

(b) CARBOXYLATED CELLULOSE NANOCRYSTAL

(c) CARBOXYLATED CHITIN NANOCRYSTAL

Fig. 2

FIG. 2 (Example 18)

## ADHESION SCORE

Mean±S.E.

*p < 0.01 (vs. SALINE , DUNNETT METHOD )

Fig. 3

FIG. 3 (Example 19)

## ADHESION SCORE

Mean±S.E.
*p < 0.01 ( vs. SALINE , DUNNETT METHOD )

Fig. 4

FIG. 4 (Example 20)

LENGTH OF ADHESION （mm）

Mean±S.E.

*p < 0.05,  **p < 0.01  (vs. SALINE , DUNNETT METHOD )

FIG. 5 (Example 21)

LENGTH OF ADHESION （mm）

Fig. 6

FIG. 6 (Example 22)

LENGTH OF ADHESION （mm）

Mean±S.E.
*p < 0.05 （vs. SALINE , DUNNETT METHOD ）

Fig. 7

FIG. 7 (Example 23)

LENGTH OF ADHESION （mm）

Mean±S.E.

*p < 0.05 （vs. SALINE , DUNNETT METHOD）

Fig. 8

FIG. 8 (Example 24)

# LENGTH OF ADHESION （mm）

Mean±S.E.
*p < 0.05 （t-TEST）

|  | SALINE | CARBOXYLATED CNF (1.2 wt%) | CARBOXYLATED CNC (1.2 wt%) |
|---|---|---|---|
|  | n=12 | n=11 | n=12 |
|  |  | EXAMPLE 4 | EXAMPLE 13 |

Fig. 9

FIG. 9 (Example 25)

# LENGTH OF ADHESION （mm）

Mean±S.E.
*p < 0.01 （vs. SALINE , DUNNETT METHOD）

| | SALINE | UNMODIFIED CHITIN NF (1.0 wt%) | CARBOXYLATED CHITIN NC (1.0 wt%) |
|---|---|---|---|
| | n=10 | n=8 | n=10 |
| | | COMPARATIVE EXAMPLE 6 | EXAMPLE 17 |

Fig. 10

FIG. 10 (Example 26)

## ADHESION SCORE

Mean±S.E.
*p < 0.05,  **p < 0.01  (vs. SALINE , DUNNETT METHOD)

## INTERNATIONAL SEARCH REPORT

| | International application No. |
|---|---|
| | PCT/JP2021/020791 |

**A. CLASSIFICATION OF SUBJECT MATTER**
A61L 31/04(2006.01)i; A61K 47/36(2006.01)i; A61K 47/38(2006.01)i
FI: A61L31/04 120; A61K47/38; A61K47/36

According to International Patent Classification (IPC) or to both national classification and IPC

**B. FIELDS SEARCHED**

Minimum documentation searched (classification system followed by classification symbols)
A61L31/04; A61K47/36; A61K47/38

Documentation searched other than minimum documentation to the extent that such documents are included in the fields searched
Published examined utility model applications of Japan       1922-1996
Published unexamined utility model applications of Japan     1971-2021
Registered utility model specifications of Japan             1996-2021
Published registered utility model applications of Japan     1994-2021

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | US 2019/0015564 A1 (SOONCHUNHYANG UNIVERSITY INDUSTRY ACADEMY COOPERATION FOUNDATION) 17 January 2019 (2019-01-17) claim 1, paragraphs [0025]-[0026], example 1, table 1, test example 8 | 1-4, 8-10, 12-18 |
| Y | | 5-7 |
| X | SULTANA, T. et al., "TEMPO oxidized nano-cellulose containing thermo-responsive injectable hydrogel for post-surgical peritoneal tissue adhesion prevention", Materials Science and Engineering: C, 2019, vol. 102, pp. 12-21., ISSN:0928-4931, abstract, fig. 1, 6 | 1-4, 8-10, 12-18 |
| Y | | 5-7 |

☒ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 July 2021 (19.07.2021) | 10 August 2021 (10.08.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2021/020791 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | 磯貝 明, バイオ系ナノファイバーの調整と高機能部材への応用展開, 繊維学会誌, 2009, vol. 65, no. 1, pp. 7-8., ISSN: 0037-9875 page 7, left column, line 21 to right column, line 25, page 8, left column, lines 1-10, fig. 1-2, (ISOGAI, Akira, "Preparation of Bio-nanofibers and Their Applications to Highly Functionalized Materials", Journal of the Society of Fiber Science and Technology, Japan) | 5-7 |
| Y | WO 2005/000374 A1 (DENKI KAGAKU KOGYO KABUSHIKI KAISHA) 06 January 2005 (2005-01-06) examples 17-18 | 1-8 |
| Y | JP 3-167201 A (HOWMEDICA INC.) 19 July 1991 (1991-07-19) claims 1, 7, 11 | 1-8 |
| Y | WO 2015/099083 A1 (NISSAN CHEMICAL INDUSTRIES, LTD.) 02 July 2015 (2015-07-02) production examples 1-3, comparative production examples 1-2, examples 1-6, comparative examples 2-3, table 2 | 1-8 |

Form PCT/ISA/210 (continuation of second sheet) (January 2015)

**INTERNATIONAL SEARCH REPORT**
Information on patent family members

International application No.

PCT/JP2021/020791

| Patent Documents referred in the Report | Publication Date | Patent Family | Publication Date |
|---|---|---|---|
| US 2019/0015564 A1 | 17 Jan. 2019 | KR 10-2019-0007826 A | |
| WO 2005/000374 A1 | 06 Jan. 2005 | US 2007/0020314 A1 examples 17-18 EP 1640026 A1 | |
| JP 3-167201 A | 19 Jul. 1991 | US 5093319 A claims 1, 7, 11 EP 426368 A2 DE 69015775 T2 ES 2066152 T3 DK 426368 T3 IE 903903 A1 AT 116555 T AU 612085 B1 CA 2028709 A1 IL 96101 DO GR 3015095 T3 KR 10-1991-0007549 A ZA 908676 B | |
| WO 2015/099083 A1 | 02 Jul. 2015 | US 2016/0325011 A1 production examples 1-3, comparative production examples 1-2, examples 1-6, comparative examples 2-3, table 2 EP 3088008 A1 TW 201538163 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015099083 A1
- WO 2010080264 A1
- US 20190015564 A
- JP 2008001728 A
- JP 2017155024 A
- WO 2018230354 A1
- WO 2018131721 A1
- WO 2014185505 A1
- JP 2017025468 A
- JP 2013249448 A
- JP HEI7101866 A, Yamauchi

### Non-patent literature cited in the description

- Intra-peritoneal prophylactic agents for preventing adhesions and adhesive intestinal obstruction after non-gynaecological abdominal surgery. *Cochrane Database Syst Rev.,* 21 January 2009, vol. 1, CD005080
- *Materials Science and Engineering C,* 2019, vol. 102, 12-21
- *ACS Sustainable Chem Eng,* 2020, vol. 8, 17800-17806
- *Biomacromolecules,* 2008, vol. 9, 192-198
- *Carbohydrate Polymers,* 2018, vol. 197, 349-358
- *Cellulose Commun,* 2016, vol. 23, 193-199
- *LWT-Food Science and Technology,* 2017, vol. 86, 318-326
- **FAN et al.** *Biomacromolecules,* 2008, vol. 9, 192-198
- **KOSAKA et al.** *Nature Medicine Letters,* 2008, vol. 14 (4), 437-441